# EUROPEAN PATENT SPECIFICATION

(11) **EP 0 741 558 B1**
(45) Date of publication and mention of the grant of the patent: **15.07.1998**
(21) Application number: 95910719.4
(22) Date of filing: 01.03.1995
(51) Int. Cl.: A61K 7/06

(54) **HAIR COSMETIC COMPOSITION**
Haarkosmetisches Präparat
COSMETIQUE CAPILLAIRE

(30) Priority: 02.03.1994 JP 32315/94; 08.12.1994 JP 304871/94
(43) Date of publication of application: 13.11.1996
(73) Proprietor: Kao Corporation, Chuo-Ku Tokyo 103 (JP)
(72) Inventor: MATSUO, Takashi, Chiba 272 (JP); YAHAGI, Kazuyuki, Tokyo 131 (JP)
(74) Representative: Hansen, Bernd, Dr. Dipl.-Chem.
(86) International application number: JP9500326
(87) International publication number: WO9523579

(56) References cited:
- EP-A- 0 426 520
- WO-A-93/08787
- GB-A- 2 098 226

## Description

The present invention relates to hair cosmetic compositions which are stable on storage and which exhibit excellent conditioning effects. The present invention also relates to methods of washing and conditioning hair with such a composition.

### Discussion of the Background:

Recently, a variety of silicone derivatives have come to be used for purposes of providing hair cosmetic compositions such as shampoos, hair rinses, and hair treatments (hair conditioners) with conditioning effects, especially for imparting smoothness to the hair after drying. Major silicone derivatives used for such purposes include water-insoluble dimethylpolysiloxanes, amino-modified silicones in O/W emulsions, and water-soluble polyether-modified silicones, of which dimethylpolysiloxanes have been widely used, because they have excellent conditioning effects.

However, dimethylpolysiloxanes have the drawback that their dispersion stability in final products, especially aqueous systems, is easily lost because of their insolubility in water, resulting in a greatly limited use with respect to the amount of incorporation, etc. Attempts have been made to enhance the dispersion stability by reducing the particle size of dimethylpolysiloxanes in the dispersion state or by using them in only very small amounts. In these cases, however, sufficient conditioning effects have not been obtained. Another problem with dimethylpolysiloxanes is that increased amounts of them cause a significant decrease in lathering ability.

In contrast, water-soluble polyether-modified silicones can be easily and stably incorporated into aqueous products. However, they have the shortcoming that they are easily washed away with very little of them remaining on the hair fibers, resulting in poor conditioning.

Thus, there remains a need for hair care compositions which have excellent stability and which provide excellent conditioning effects.

### SUMMARY OF THE INVENTION

Accordingly, it is one object of the present invention to provide novel hair care compositions.

It is another object of the present invention to provide novel hair care compositions which are stable on storage.

It is another object of the present invention to provide a method for washing hair by applying such a hair care composition to the hair.

It is another object of the present invention to provide a method of conditioning hair by applying such a hair care composition to the hair.

These and other objects, which will become apparent during the following detailed description have been achieved by the inventors' discovery that the combined use of organopolysiloxanes modified with a structure having an acidic group or a salt thereof and a water-soluble cationic polymer in specified amounts is effective for obtaining hair cosmetic compositions which are stable during storage, which persist on hair fibers due to an aggregation of the two components, and which thereby exhibit excellent conditioning effects.

Accordingly, the present invention provides hair cosmetic compositions which comprise the following components (A) and (B):
(A) 0.1 to 20% by weight, based on the total weight of the composition, of an organopolysiloxane having at least one structure containing an acidic group or a salt thereof in its molecule; and
(B) 0.01 to 5% by weight, based on the total weight of the composition, of a water-soluble cationic polymer.

### DETAILED DESCRIPTION OF THE PREFERRED EMBODIMENTS

The "organo(poly)siloxane" in component (A) of the present invention is intended to encompass organopolysiloxanes of from high-molecular weight (number average molecular weight of about 1,000,000) to low-molecular weight (number average molecular weight of about 200). Thus, in the context of the present invention, the term "organo(poly)siloxanes" includes disiloxanes.

Examples of the "salt" in component (A) of the invention include alkali metal salts, ammonium salts, mono-, di-, tri-, and tetra(C₁₋₄-alkyl)ammonium salts, mono-, di-, tri-, and tetra(C₂₋₄-alkanol)ammonium salts, pyridinium salts, etc. Examples of alkali metals for the alkali metal salts include Li, Na, K, Rb, and Cs.

Examples of the acidic group or the salt of component (A), i.e., the organopolysiloxane having at least one structure containing an acidic group or a salt thereof in its molecule (hereinafter referred to as "acidic group-modified organopolysiloxane"), include a phosphoric group, a phosphate group, a carboxylic group, a carboxylate group, a sulfuric group, a sulfate group, a sulfonic group, and a sulfonate group.

Of the acidic group-modified organopolysiloxanes, examples of the organopolysiloxanes which are modified with a phosphoric group or a phosphate (organopolysiloxanes (A-1)) include phosphoric-modified organopolysiloxanes or their salts having a structural unit in their molecules represented by the following formula (1): 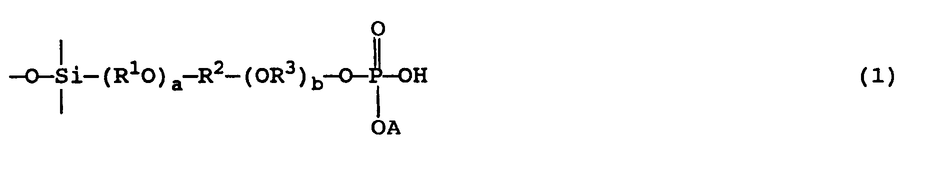 wherein R¹ and R³ each independently represent a C₂₋₂₀ linear or branched alkylene group, R² represents a C₁₋₅₀ linear or branched alkylene group which may have a hydroxyl group, A represents a hydrogen atom, a C₁₋₂₂ linear or branched alkyl or alkenyl group, or the group, 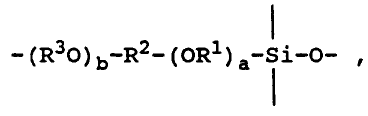 a represents an integer of 0 or 1, and b represents a number from 0 to 200 inclusive.

In the above formula (1), examples of the C₂₋₂₀ linear or branched alkylene group represented by R¹ and R³ include ethylene, propylene, trimethylene, butylene, pentamethylene, hexamethylene, heptamethylene, octamethylene, nonamethylene, decamethylene, undecamethylene, dodecamethylene, tridecamethylene, tetradecamethylene, pentadecamethylene, hexadecamethylene, heptadecamethylene, octadecamethylene, nonadecamethylene, eicosamethylene, etc. Of these, C₂₋₄ linear or branched alkylene groups are especially preferred. a is preferably 0, and b is preferably from 0 to 15.

Examples of the C₁₋₅₀ linear or branched alkylene group which may have a hydroxyl group represented by R² include methylene, ethylene, trimethylene, propylene, 1-methylpropylene, butylene, pentamethylene, 3-methylbutylene, 1,1-dimethylpropylene, hexamethylene, octamethylene, decamethylene, undecamethylene, 3-methylpentane-1,5-diyl, 2-ethylhexane-1,6-diyl, 3,7-dimethyloctane-1,8-diyl, 3,7-dimethyloctane-3,8-diyl, 2-hydroxyethylene, 2-hydroxybutylene, and 2-hydroxyoctamethylene. Of these, C₂₋₁₅ alkylene groups which may have a hydroxy group, particularly C₃₋₁₂ alkylene groups which may have a hydroxy group, are especially preferred.

Examples of the C₁₋₂₂ alkyl or alkenyl represented by A include methyl, ethyl, propyl, isopropyl, butyl, hexyl, pentyl, t-butyl, octyl, nonyl, decyl, undecyl, dodecyl, tridecyl, tetradecyl, pentadecyl, hexadecyl, heptadecyl, octadecyl, nonadecyl, eicosyl, heneicosyl, docosyl, ethenyl, propenyl, butenyl, pentenyl, hexenyl, heptenyl, octenyl, nonenyl, decenyl, dodecenyl, undecenyl, tridecenyl, tetradecenyl, pentadecenyl, hexadecenyl, heptadecenyl, octadecenyl, nonadecenyl, eicosenyl, heneicosenyl, and docosenyl.

The phosphoric modified organo(poly)siloxanes (A-1) are not particularly limited as long as they contain the structural unit of formula (1) in their molecules. Preferable examples include those represented by the following formulae (A-1a) to (A-1c).
(A-1a) Phosphate-modified organo(poly)siloxanes of formula (2): 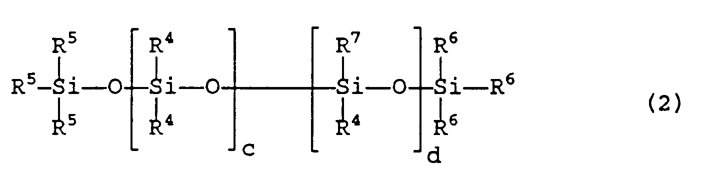 wherein the R⁴'s are the same as or different from each other and each represent a C₁₋₂₂ alkyl group, a C₁₋₂₂ alkoxy group, or a phenyl group; R⁵'s, R⁶'s, and R⁷ are the same as or different from each other, at least one group of which is represented by the following formula (3): 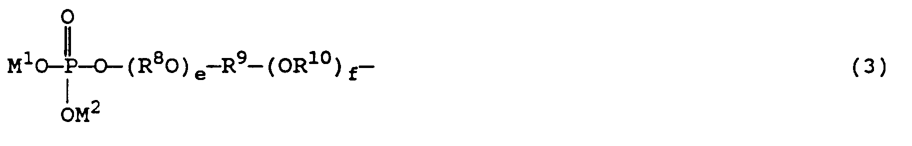 wherein R⁸ and R¹⁰ are a C₂₋₂₀ alkylene group, R⁹ is a C₁₋₅₀ linear or branched alkylene group which may have a hydroxyl group, e is a number from 0 to 200 inclusive, f is 0 or 1, and M¹ and M² are hydrogen, alkali metal, ammonium, mono-, di-, tri-, or tetra(C₁₋₄-alkyl)ammonium, mono-, di-, tri-, or tetra(C₂₋₄-alkanol)ammonium, or pyridinium, and the remaining R⁵'s, R⁶'s and R⁷ groups are C₁₋₂₂ alkyl, C₁₋₂₂ alkoxy, phenyl, or a group of formula (4):

   HO-(R⁸O)ₑ-R⁹-(OR¹⁰)_{f}- (4)

   wherein R⁸, R⁹, R¹⁰, e, and f have the same meaning as defined above; and c and d are numbers from 0 to 1,000 inclusive.
   The group R⁴ of formula (2) is C₁₋₂₂ alkyl, C₁₋₂₂ alkoxy, or phenyl. Of these, C₁₋₆ alkyl or C₁₋₆ alkoxy, particularly methyl, are preferred. Although c and d in formula (2) are a number from 0 to 1,000 inclusive, they are preferably 0 to 150, more preferably 0 to 50, and particularly preferably from 0 to 20. The sum of c and d is preferably 2 to 1,000. The group R⁹ of formula (3) is preferably a group of C₂₋₁₅, more preferably, C₃₋₁₂. R⁸ and R¹⁰ are preferably C₂₋₄ alkylene. e is preferably from 0 to 15 inclusive, and f is preferably 0. M¹ and M² are preferably hydrogen or alkali metal.
(A-1b) Monoalkylphosphate-modified organo(poly)siloxanes of formula (5): 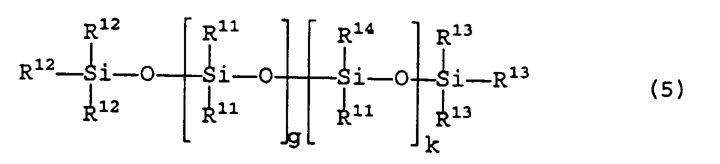 wherein the R¹¹'s are the same as or different from each other and each represent a C₁₋₂₂ alkyl, C₁₋₂₂ alkoxy group, or a phenyl group; R¹²'s, R¹³'s, and R¹⁴ are the same as or different from each other, at least one group of which is represented by the following formula (6): 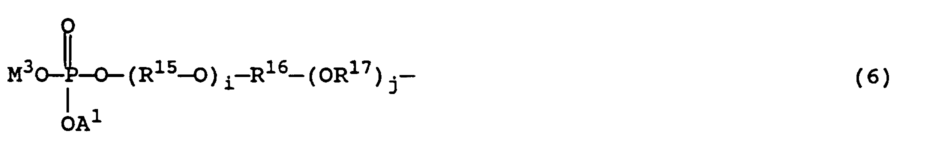 wherein R¹⁵ and R¹⁷ are a C₂₋₄ alkylene group, R¹⁶ is a C₁₋₅₀ linear or branched alkylene group which may have a hydroxyl group, j is a number from 0 to 200 inclusive, i is 0 or 1, M³ is hydrogen, alkali metal, ammonium, mono-, di-, tri-, or tetra(C₁₋₄-alkyl)ammonium, mono-, di-, tri-, or tetra(C₂₋₄-alkanol)ammonium, or pyridinium, A¹ is a C₁₋₂₂ linear or branched alkyl or alkenyl group, and the remaining R¹²'s, R¹³'s, and R¹⁴ groups are C₁₋₂₂ alkyl, C₁₋₂₂ alkoxy, phenyl, or a group of formula (7):

   HO-(R¹⁵O)ᵢ-R¹⁶-(OR¹⁷)ⱼ- (7)

   wherein R¹⁵, R¹⁶, R¹⁷, i, and j have the same meaning as defined above; and g and k are numbers from 0 to 1,000 inclusive.
   The group R¹¹ of formula (5) is C₁₋₂₂ alkyl, C₁₋₂₂ alkoxy, or phenyl. Of these, C₁₋₆ alkyl or C₁₋₆ alkoxy, particularly methyl, are preferred. Although g and k in formula (5) are a number from 0 to 1,000 inclusive, they are preferably 0 to 150, more preferably 0 to 50, and particularly preferably from 0 to 20. The sum of g and k is preferably 2 to 1,000. The group R¹⁶ of formula (6) is preferably a group of C₂₋₁₅, more preferably, C₃₋₁₂. R¹⁵ and R¹⁷ are preferably C₂₋₄ alkylene. i is preferably from 0 to 15 inclusive, and j is preferably 0. M³ is preferably hydrogen or alkali metal. A¹ is preferably C₁₋₁₈ linear or branched alkyl.
(A-1c) Phosphoric diester-modified organo(poly)siloxanes of formula (8): 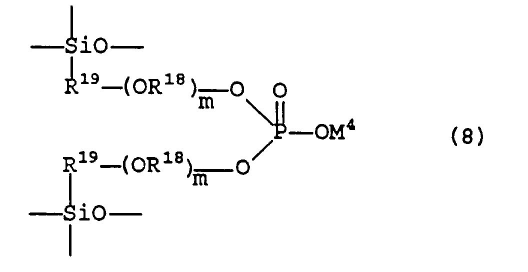 wherein R¹⁸ is C₂₋₂₀ alkylene, R¹⁹ is C₁₋₅₀ linear or branched alkylene which may be substituted by hydroxyl, m is a number from 0 to 200 inclusive, and M⁴ is hydrogen, alkali metal, ammonium, mono-, di-, tri-, or tetra(C₁₋₄-alkyl)ammonium, mono-, di-, tri-, or tetra(C₂₋₄-alkanol)ammonium, or a pyridinium salt.

The group R¹⁸ of formula (8) is a C₂₋₂₀ alkylene group as described above. Preferably, R¹⁸ is butylene, pentamethylene, hexamethylene, heptamethylene, octamethylene, nonamethylene, decamethylene, undecamethylene, and dodecamethylene. The group R¹⁹ is a C₁₋₅₀ linear or branched alkylene group which may be substituted by hydroxyl as described above. Preferably, R¹⁹ is methylene, ethylene, propylene, butylene, pentamethylene, hexamethylene, and octamethylene. Although m is a number from 0 to 200 inclusive, it is preferably 0 to 20. M⁴ is preferably alkali metal, and particularly, Na or K.

These phosphoric diester-modified organo(poly)siloxanes (A-1c) are not particularly limited as long as they contain the structural unit of formula (8) in their molecules. The types of linkage of the structure are not particularly limited, either. For example, the structural unit (8) may be linked to a silicone chain in a linear manner, networking manner, ring-forming manner, or in a combination of these. Moreover, structural unit (8) and a silicone chain may be linked to each other at random or in blocks.

Among the various types of linkage structures, the structure represented by formula (9) is particularly preferred. 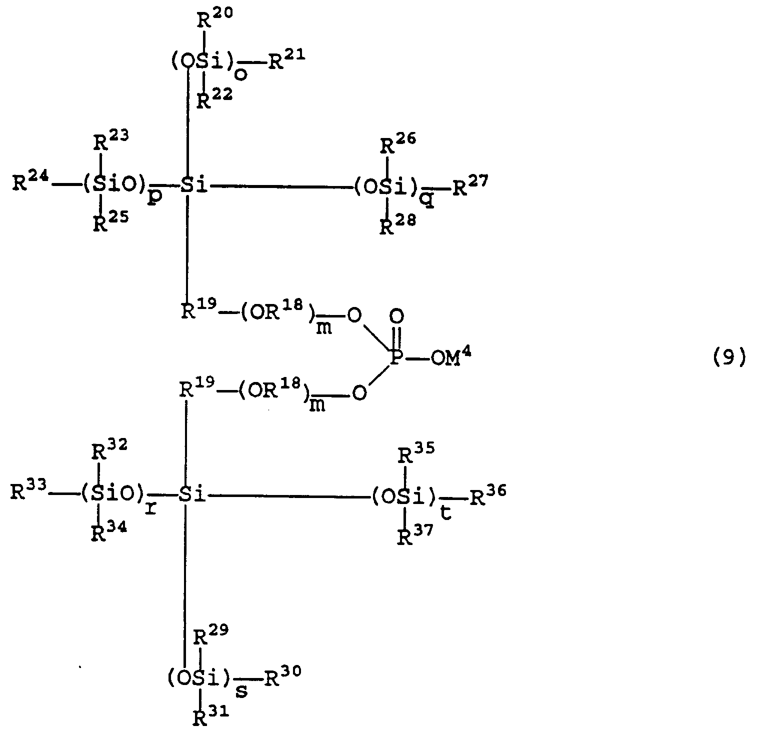 wherein R¹⁸, R¹⁹, m, and M⁴ have the same meanings as defined above; R²⁰ to R³⁷ are C₁₋₂₂ alkyl, C₁₋₂₂ alkoxy, or phenyl, and wherein R²⁴ and R³³ may together be a divalent oxygen atom; and o, p, q, r, s, and t are each a number from 0 to 1,000, inclusive. The sum of o, p and q, and the sum of r, s and t are both preferably 2 to 1,000. The sum of o, p, q, r, s and t is preferably 3 to 1,000.

The phosphate-modified organo(poly)siloxanes (A-1) may be prepared by the method described in Japanese Patent Application Laid-open (kokai) No. 6-16684. They are however preferably prepared by one of the following processes 1 to 3:

### Process 1

Among the aforementioned phosphoric-modified organo(poly)siloxanes (A-1), the phosphate-modified organopolysiloxanes (2) of formula (A-1a) can be prepared, for example, by first reacting phosphorus oxyhalide with alcohol-modified organo(poly)siloxane having at least one group of formula (4) bonded to a silicon atom in the molecular chain, and then hydrolyzing the bond between phosphorus and halogen. The thus-obtained organo(poly)siloxanes (2) contain a group of formula (3) which is bound to a silicon atom in the molecular chain.

This process will next be described in detail. In the first step, phosphorus oxyhalide is reacted with the alcohol-modified organo(poly)siloxane having at least one group of formula (4) bonded to a silicon atom in the molecular chain (hereinafter simply referred to as the alcohol-modified organo(poly)siloxane) in the presence or absence of a solvent.

The alcohol-modified organo(poly)siloxanes which are used in the present invention may be of any type as long as they contain a group (4) which is linked to at least one silicon atom present in the molecular chain. The bonding site is not particularly limited. Therefore, alcohol-modified organo(poly)siloxanes categorized as a side-chain type, two-terminal type, one-terminal type, T-structure type, etc. depending on the bonding site of a specific substituent are all usable. Specific examples of the structures of the alcohol-modified organo(poly)siloxanes include those represented by the following formulae. 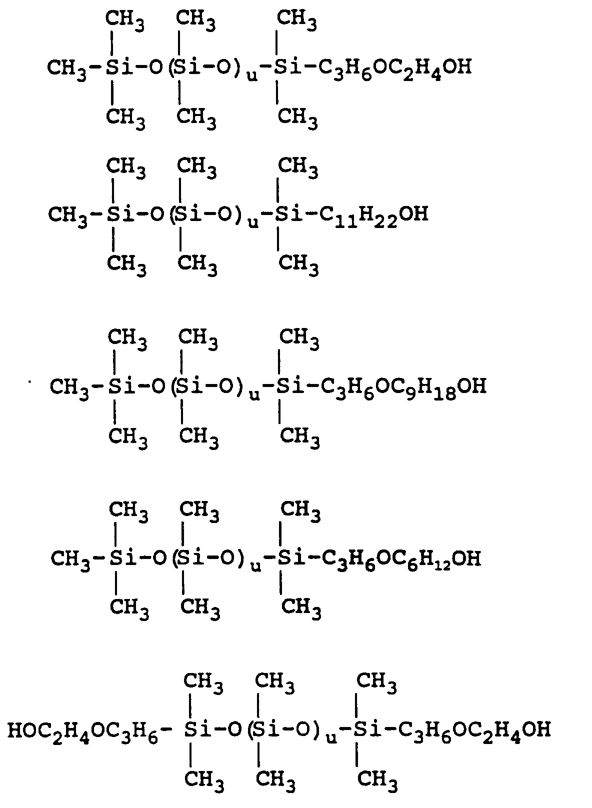 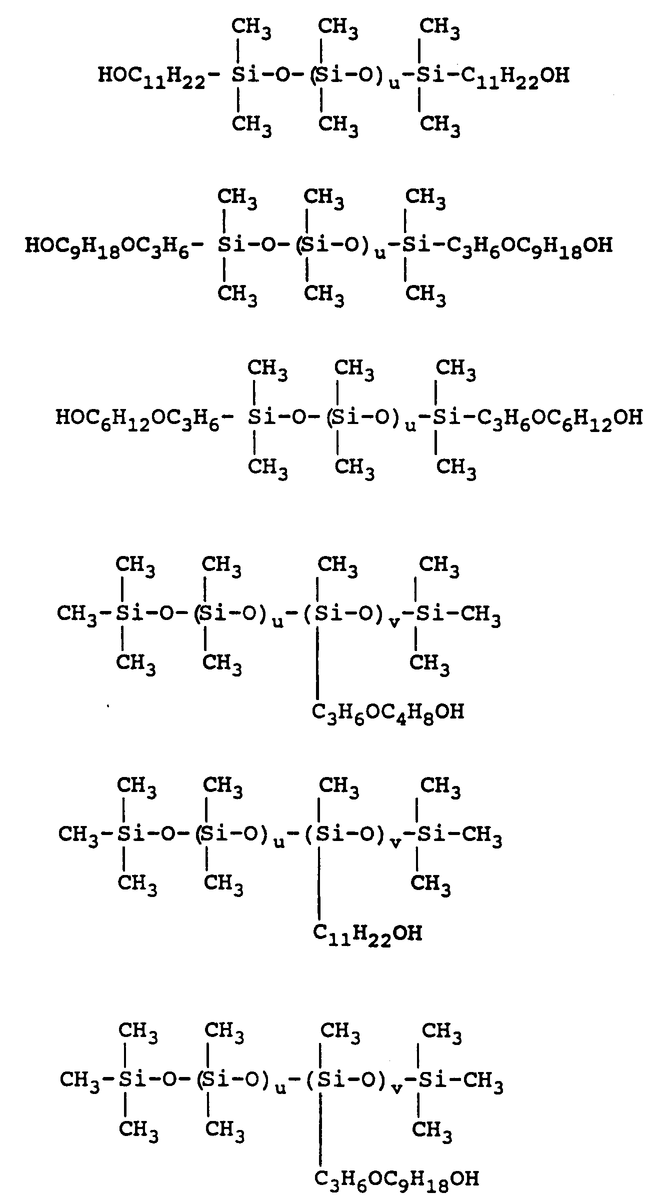 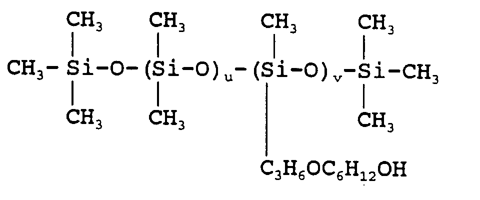 wherein u and v are each a number from 0 to 1,000 inclusive. Each of u and v is preferably 0 to 1,000, suitably 1 to 500.

The above-described alcohol-modified organo(poly)siloxanes may be prepared by known methods or they may be commercially purchased and used as they are. Examples of the commercially available products include X-22-170, X-22-170A, X-22-170B, X-22-170D, X-22-160AS, KF6001, KF6002, KF6003, X-22-176B, X-22-176D, X-22-4015, KF6005, KF6007, KF6015, KF353A, KF354A, KF355A (products of Shin'etsu Kagaku), TSL-9105, TSF4705, TSF4751, XF42-220, XF42-811, XF42-831 (products of Toshiba Silicone), and PS197 and PX101 (products of Chisso).

Examples of the phosphorus oxyhalide which is to be reacted with the above-described alcohol-modified organo(poly)siloxanes include phosphorus oxychloride and phosphorus oxybromide, with phosphorus oxychloride being particularly preferred.

The conditions of the reaction between the alcohol-modified organo(poly)siloxane and the phosphorus oxyhalide are not particularly limited. For example, they may be independently dissolved in suitable solvents, and then may be mixed to cause a reaction.

Examples of the solvent for dissolving the alcohol-modified organo(poly)siloxane include tetrahydrofuran, methylene chloride, toluene, and diethyl ether. The amount of the solvent is not more than 10 times and preferably not more than 3 times the weight of the alcohol-modified organo(poly)siloxane. Examples of the solvent for dissolving the phosphorus oxyhalide include tetrahydrofuran, methylene chloride, toluene, and diethyl ether. The amount of the solvent is not more than 20 times and preferably not more than 10 times the weight of the phosphorus oxyhalide.

The ratio of the amount of alcohol-modified organo(poly)siloxane to that of phosphorus oxyhalide is not particularly limited. Preferably, the alcohol-modified organo(poly)siloxane and the phosphorus oxyhalide are mixed at a ratio of phosphorus oxyhalide : alcohol-modified organo(poly)siloxane being 0.5:1 to 2:1, more preferably 0.9:1 to 1.5:1, on an equivalent basis.

The method of mixing the alcohol-modified organo(poly)siloxane and the phosphorus oxyhalide is not particularly limited. For example, a solution of alcohol-modified organo(poly)siloxane may be added dropwise to a solution of phosphorus oxyhalide with stirring if desired.

The manner of addition is not particularly limited. For example, the total amount may be poured at a time, or may be divided and added portionwise. Alternatively, the alcohol-modified organo(poly)siloxane may be added dropwise. During addition, the temperature of the reaction system is preferably maintained at -50 to 10°C and more preferably at -30 to 0°C. After completion of the reaction, the reaction is cured for 1 to 5 hours within the above temperature ranges. In order to neutralize the by-product hydrochloric acid, tertiary amines such as triethylamine, tributylamine, pyridine, or N-methylmorpholine may be added.

Next, in order to hydrolyze the phosphorus - halogen bonds in the reaction product produced in the preceding step, an alkaline solution is applied in a manner as described above.

Examples of the alkaline solution include aqueous solutions of NaOH, KOH, ammonia, mono-, di-, tri-, or tetra(C₁₋₄-alkyl)amine, mono-, di-, tri-, or tetra(C₂₋₄-alkanol)amine, with NaOH and KOH being particularly preferred. The concentration of the alkaline solution is not particularly limited. It is suitably controlled depending on the concentrations of the alcohol-modified organo(poly)siloxane and phosphorus oxyhalide. The temperature of the reaction system is preferably set and maintained within the ranges as described above. The reaction time is preferably from 1 to 15 hours.

When the reaction is completed, the phosphate-modified organo(poly)siloxane (2) produced is separated from the reaction system. If necessary, the separated phosphate-modified organo(poly)siloxane may be subjected to further purification processes, which are not particularly limited. For example, the following three methods may be used. 1) Water, a nonhydrophilic solvent such as butanol and toluene, and a demulsifier such as ethanol and 2-propanol are added to the reaction solution, and the resulting mixture is stirred. Subsequently, the mixture is allowed to stand for phase separation, after which the aqueous phase containing excess phosphate and by-produced inorganic or organic salts are removed. 2) The solvent is removed from the reaction solution, and the residue is washed with water to remove excess phosphate, etc. Alternatively, the residue is dissolved in a solvent such as ethanol, propanol, or toluene, and excess phosphate and the like materials which settle are removed by filtration. 3) If the produced phosphate-modified organo(poly)siloxane is insoluble in an organic solvent, a hydrophilic solvent such as ethanol and acetone is added to the aqueous phase collected to precipitate the target compound.

### Process 2

The monoalkylphosphate-modified organopolysiloxanes (5) of formula (A-1b) can be prepared, for example, by first reacting phosphorus oxyhalide with alcohol-modified organo(poly)siloxane having at least one group of formula (7) bonded to a silicon atom in the molecular chain:

HO-(R¹⁵O)ᵢ-R¹⁶-(OR¹⁷)ⱼ- (7)

wherein R¹⁵, R¹⁶, R¹⁷, i, and j have the same meanings as defined hereinbefore, subsequently reacting with an alcohol represented by A¹-OH wherein A¹ has the same meaning as defined hereinbefore, and then hydrolyzing the bond between phosphorus and halogen. The thus-obtained organo(poly)siloxanes (5) contain a group of formula (6): 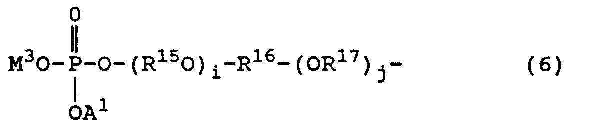 wherein R¹⁵, R¹⁶, R¹⁷, i, j, A¹ and M³ have the same meanings as defined hereinbefore, which group is bound to a silicon atom in the molecular chain.

The reaction between the alcohol-modified organo(poly)siloxane and phosphorus oxyhalide is carried out in a manner similar to that described for the preparation of phosphate-modified organo(poly)siloxanes (2) described in Process 1.

The reaction product thus obtained is reacted with an alcohol represented by A¹-OH wherein A¹ has the same meaning as defined above.

Examples of the alcohol represented by A¹-OH include methanol, ethanol, 1-propanol, 2-propanol, 1-butanol, 2-butanol, tert-butanol, 1-pentanol, 1-hexanol, 1-heptanol, 1-octanol, 1-nonanol, 1-decanol, 1-undecanol, 1-dodecanol, 1-tetradecanol, 1-hexadecanol, 1-octadecanol, 2-ethylhexanol, 2-hexyldecanol, 2-heptylundecanol, 1-oleylalcohol, tetradecafluorohexylethyl alcohol, and heptadecafluorooctylethyl alcohol.

The manner of addition of A¹-OH is not particularly limited. For example, the total amount may be added at once, or it may be divided and added portionwise. Alternatively, the alcohol-modified organo(poly)siloxane may be added dropwise. During addition, the temperature of the reaction system is preferably maintained at -50 to 10°C and more preferably at -30 to 0°C. After completion of the reaction, the reaction is cured for 1 to 5 hours within the above temperature ranges.

It is preferred that the alcohol of A¹-OH be used in an amount of from 0.8 to 1.2 equivalents with respect to the phosphorus oxyhalide.

In order to hydrolyze the phosphorus - halogen bonds remaining in the reaction product produced in the preceding step, a method similar to that described in Process 1 may be used.

### Process 3

The phosphoric diester-modified organo(poly)siloxanes of formula (A-1c) can also be prepared in accordance with Process 1. It is however preferred that the reaction ratio of alcohol-modified organo(poly)siloxane to phosphorus oxyhalide be roughly 2:1 on a molar basis, and the reaction temperature be controlled to carry out a two-step reaction. In detail, alcohol-modified organo(poly)siloxane is reacted with phosphorus oxyhalide in the presence of a tertiary amine such as triethylamine, tributylamine, pyridine, or N-methylmorpholine in amounts of 1 to 3 times the total weight of the starting materials in a solvent capable of dissolving both of the starting materials such as tetrahydrofuran, methylene chloride, or toluene in amounts of 0 to 20 times the total weight of the starting materials at a temperature ranging from 0 to 30°C for 1 to 30 hours. Preferably, the reaction is initially carried out at a temperature of from -20 to -30°C for 1 to 5 hours to produce a monoester, after which the reaction temperature is shifted to the temperature range of from 0 to 15°C to continue the reaction for 1 to 30 hours more to produce a diester. Subsequently, an aqueous alkaline solution in an amount of 3 to 5 times the equivalents of the phosphorus oxyhalide used for the reaction is added to the reaction and the P-X bonds generated are hydrolyzed at -30 to 0°C for 2 to 12 hours.

The reaction ratio between the starting materials, i.e., the ratio of alcohol-modified organo(poly)siloxane to phosphorus oxyhalide is not particularly limited, and it is suitably determined in accordance with the degree of modification of the target compound, modified organo(poly)siloxane. For example, given that the number of the hydroxyl groups contained in the starting alcohol-modified organo(poly)siloxane is J, and all the hydroxyl groups in the number of J are desired to be modified, the amount of the phosphorus oxyhalide is from 0.2 to 0.8 X J, preferably from 0.3 to 0.6 X J, per mole of the alcohol-modified organo(poly)siloxane.

Among the acidic group-modified organopolysiloxanes described above, examples of the organopolysiloxanes modified with a carboxylic group or a carboxylate-containing group (A-2) include carboxylic acid-modified organopolysiloxanes having a structural unit in their molecules represented by the following formula (10): 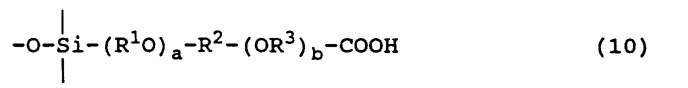 wherein R¹, R², R³, a, and b have the same meanings as defined above, and their salts.

Preferable examples of the carboxylic acid-modified organopolysiloxanes (A-2) include those represented by the following formula (11): 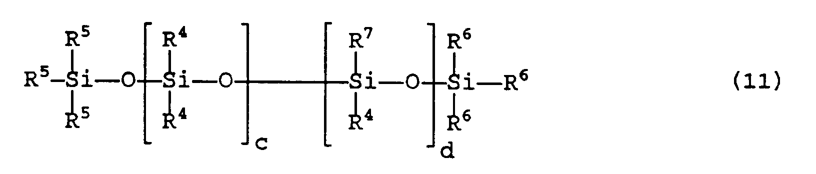 wherein the R⁴'s are the same or different from each other and each represent a C₁₋₂₂ alkyl, C₁₋₂₂ or alkoxy group, or a phenyl group, and R⁵'s, R⁶'s, and R⁷ are the same as or different from each other, at least one group of which is represented by the following formula (12):

-(R¹⁰O)_{f}-R⁹-(OR⁸)ₑ-COOM¹ (12)

wherein R⁸ and R¹⁰ are a C₂₋₂₀ alkylene group, R⁹ is a C₁₋₅₀ linear or branched alkylene group which may have a hydroxyl group, M¹ is hydrogen, alkali metal, ammonium, mono-, di-, tri-, or tetra(C₁₋₄-alkyl)ammonium, mono-, di-, tri-, or tetra(C₂₋₄-alkanol)ammonium, or pyridinium, e is a number from 0 to 200 inclusive, and f is 0 or 1; and the remaining groups are C₁₋₂₂ alkyl, C₁₋₂₂ alkoxy, phenyl, or a group of formula (13):

-(R¹⁰O)_{f}-R⁹-(OR⁸)ₑ-OH (13)

wherein R⁸, R⁹, R¹⁰, e, and f have the same meanings as defined above; and c and d are numbers from 0 to 1,000 inclusive. The sum of c and d is preferably 2 to 1,000.

Preferable examples of the R¹ to R⁷, a, b, c, d, e, f, and M¹ are the same as described hereinbefore.

The carboxylic acid-modified organo(poly)siloxanes (A-2) which are used in the present invention can be prepared in accordance with a method described, for example, in Silicone Handbook (Kunio ITOH, published by Nikkan Kogyo Shinbun, pp. 166-167 (1990)).

Among the above-described acidic group-modified organopolysiloxanes, examples of the organopolysiloxanes modified with a sulfuric group or a sulfate-containing group (A-3) include sulfuric acid-modified organopolysiloxanes having a structural unit in their molecules represented by the following formula (14): 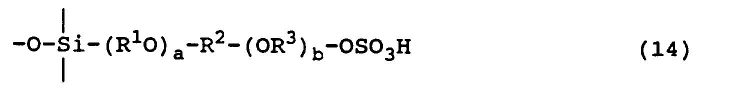 wherein R¹, R², R³, a, and b have the same meanings as defined above, and their salts.

Preferable examples of the sulfuric acid-modified organopolysiloxanes (A-3) include those represented by the following formula (15): 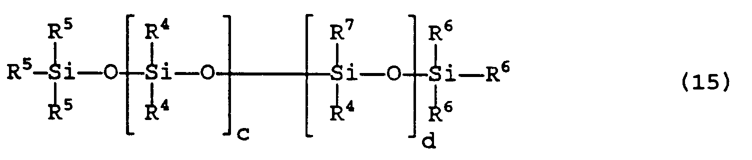 wherein the R⁴'s are the same or different from each other and each represent a C₁₋₂₂ alkyl, C₁₋₂₂ alkoxy group, or a phenyl group, and R⁵'s, R⁶'s, and R⁷ are the same as or different from each other, at least one group of which is represented by the following formula (16):

-(R¹⁰O)_{f}-R⁹-(OR⁸)ₑ-OSO₃M¹ (16)

wherein R⁸ and R¹⁰ are a C₂₋₂₀ alkylene group, R⁹ is a C₁₋₅₀ linear or branched alkylene group which may have a hydroxyl group, M¹ is hydrogen, alkali metal, ammonium, mono-, di-, tri-, or tetra(C₁₋₄-alkyl)ammonium, mono-, di-, tri-, or tetra(C₂₋₄-alkanol)ammonium, or pyridinium, e is a number from 0 to 200 inclusive, and f is 0 or 1; and the remaining groups are C₁₋₂₂ alkyl, C₁₋₂₂ alkoxy, phenyl, or a group of formula (17):

-(R¹⁰O)_{f}-R⁹-(OR⁸)ₑ-OH (17)

wherein R⁸, R⁹, R¹⁰, e, and f have the same meanings as defined above; and c and d are numbers from 0 to 1,000 inclusive. The sum of c and d is preferably 2 to 1,000.

Preferable examples of the R¹ to R⁷, a, b, c, d, e, f, and M¹ are the same as described hereinbefore.

The above-described sulfuric acid-modified organo(poly)siloxanes (A-3) can be prepared, for example, by the following process.

In short, when an alcohol-modified organo(poly)siloxane having at least one group represented by formula (16) bonded to a silicon atom in the molecular chain is reacted with a sulfation agent, a sulfate-modified organo(poly)siloxane having at least one group of formula (17) bound to a silicon atom in the molecular chain is prepared.

This process will next be described in detail. Firstly, a sulfation agent is reacted with alcohol-modified organo(poly)siloxane having at least one group of formula (16) bonded to a silicon atom in the molecular chain (hereinafter simply referred to as the alcohol-modified organo(poly)siloxane) in the presence or absence of a solvent.

The alcohol-modified organo(poly)siloxanes which are used in the present invention may be of any type as long as they contain a group (16) which is linked to at least one silicon atom present in the molecular chain. The bonding site is not particularly limited. Therefore, alcohol-modified organo(poly)siloxanes categorized as a side-chain type, two-terminal type, one-terminal type, T-structure type, etc. depending on the bonding site of a specific substituent are all usable. Specific examples of the structures of the alcohol-modified organo(poly)siloxanes include those listed in the above-described Process 1.

Examples of the sulfation agents which are reacted with the alcohol-modified organo(poly)siloxanes include a compound selected from the group consisting of chlorosulfonic acid, sulfurous acid, sulfamic acid, and adducts of these acids and a Lewis base. Of these, chlorosulfonic acid is particularly preferred in view that the reaction proceeds under mild conditions.

The conditions of reaction between the alcohol-modified organo(poly)siloxane and the sulfation agent are not particularly limited. For example, they may be independently dissolved in suitable solvents, and then may be mixed to cause a reaction.

Examples of the solvent for dissolving the alcohol-modified organo(poly)siloxane include tetrahydrofuran, methylene chloride, toluene, and diethyl ether. The amount of the solvent is not more than 10 times and preferably not more than 3 times the weight of the alcohol-modified organo(poly)siloxane. Examples of the solvent for dissolving the sulfation agent include tetrahydrofuran, methylene chloride, toluene, and diethyl ether. The amount of the solvent is not more than 20 times and preferably not more than 10 times the weight of the sulfation agent.

The ratio of the amount of alcohol-modified organo(poly)siloxane to that of the sulfation agent is not particularly limited. Preferably, the alcohol-modified organo(poly)siloxane and the sulfation agent are mixed at a ratio of sulfation agent : alcohol-modified organo(poly)siloxane being 0.5:1 to 2:1, more preferably 0.9:1 to 1.5:1, on an equivalent basis.

The method of mixing the alcohol-modified organo(poly)siloxane and the sulfation agent is not particularly limited. For example, a solution of a sulfation agent may be added dropwise to a solution of alcoholmodified organo(poly)siloxane with stirring if necessary.

The manner of addition is not particularly limited. For example, the total amount may be added at once, or it may be divided and added portionwise. Alternatively, the sulfation agent may be added dropwise. During the addition, the temperature of the reaction system is preferably maintained at -50 to 20°C and more preferably at -30 to 10°C. After completion of the reaction, the reaction is cured for 1 to 5 hours within the above temperature ranges. Since the siloxane bonds are easily cleaved when the reaction system is acidic, it is preferred that a basic compound such as an alkali metal hydroxide, alkali metal carbonate, and an amine, preferably a tertiary amine such as triethylamine, tributylamine, pyridine, or N-methylmorpholine may be added.

If sulfate-modified organo(poly)siloxanes of formula (15) in which M¹ is ammonium, alkylamine or alkanolamine are prepared, the reaction may be terminated after completion of the above step. However, if those in which M¹ is an alkali metal are prepared, an alkaline solution is added to the reaction product obtained in the above step in a similar manner as described above to cause a reaction.

Examples of the alkaline solution include aqueous solutions of NaOH, KOH, ammonia, mono-, di-, tri-, or tetra(C₁₋₄-alkyl)amine, pyridine, and mono-, di-, tri-, or tetra(C₂₋₄-alkanol)amine, and alcoholates such as sodium methylate, sodium ethylate, potassium ethylate, and potassium methylate. Particularly, aqueous solutions of NaOH and KOH, sodium methylate, and potassium methylate are particularly preferred. The concentration of the alkaline solution is not particularly limited, and it is suitably controlled depending on the concentrations of the alcohol-modified organo(poly)siloxane and the sulfation agent. The temperature of the reaction system is preferably set and maintained within the ranges as described above.

When the reaction is completed, the sulfate-modified organo(poly)siloxane (A-3) produced is separated from the reaction system. If necessary, the separated organo(poly)siloxane containing a sulfuric ester may be subjected to further purification processes, which are not particularly limited. For example, the following two methods may be used. 1) The solvent is distilled off from the reaction solution, and the residue is dissolved in a non-polar solvent such as chloroform, after which the by-product inorganic salt is removed by filtration. 2) The solvent is removed from the reaction solution, and a solvent such as hexane is added to the residue. The precipitate is collected by filtration, and unreacted and excess alcohol-modified organo(poly)siloxanes are removed.

The acidic group-modified organo(poly)siloxanes of component (A) or their salts are used singly or in combinations of two or more. They are preferably incorporated into the present hair cosmetic compositions in amounts from 0.1 to 20% by weight (hereinafter referred to simply as %), based on the total weight of the composition. When they are incorporated in amounts of 0.5 to 10%, particularly 0.5 to 5%, stable compositions can be obtained, and excellent smoothness can be imparted to the hair after drying. If the hair cosmetic compositions of the present invention are shampoos, it is preferred that the amount of the acidic group-modified organo(poly)siloxanes to be incorporated therein be from 0.1 to 10%, particularly from 0.5 to 5%, based on the total weight of the composition, in order to maximize the smoothness of the hair after drying and the stability of the resulting shampoo compositions.

The water-soluble cationic polymers of component (B) according to the present invention are preferably those which contain a polymer chain linked to an amino group or an ammonium group, or those which contain dimethyldiallylammonium halide as a structural unit. Specific examples of the water-soluble cationic polymers include cationic celluloses, cationic starches, cationic guar gums, homopolymers of diallyl quaternary ammonium salts, diallyl quaternary ammonium salt/acrylic amide copolymers, and quaternary polyvinyl pyrrolidones.

Preferable examples of the cationic celluloses include the compounds of formula (18): 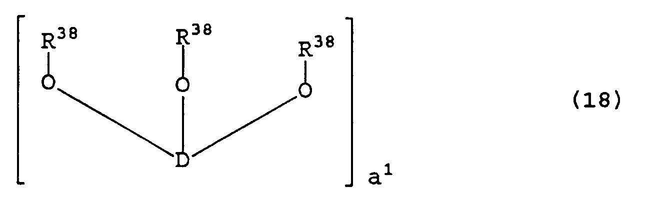 wherein D represents a residue of an anhydroglucose unit, a₁ represents a number from 50 to 20,000 inclusive, and the R³⁸'s each represent the substituent of formula (19): 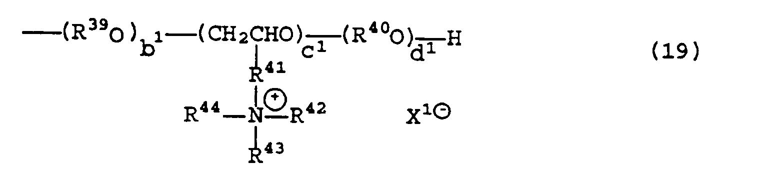 wherein R³⁹ and R⁴⁰ each independently represent C₂₋₃ alkylene, b¹ is a number from 0 to 10 inclusive, c¹ is a number from 0 to 3 inclusive, d¹ is a number from 0 to 10 inclusive, R⁴¹ is C₁₋₃ alkylene or C₁₋₃ hydroxyalkylene, R⁴², R⁴³, and R⁴⁴ are the same or different from each other and, present alkyl, aryl (phenyl), or aralkyl which have carbon atoms of 10 or less and which may form a heterocyclic ring with the nitrogen atom of the formula, and X¹ represents an anion such as chloride, bromide, iodide, a sulfuric ion, a sulfonic ion, a methylsulfuric ion, a phosphoric ion, a nitric ion, etc.

The cationic substitution degree of these cationic cellulose derivatives, or the average number of c¹ per unit of anhydroglucose is preferably 0.01 to 1, and more preferably 0.02 to 0.5. The sum of b¹ and d¹ is from 1 to 3 on average. Cationic substitution degrees of less than 0.01 are just insufficient. Whereas, although the cationic substitution degree may be higher than 1, degrees less than or equal to 1 are particularly preferred in view of the yield of the reaction. The preferable number average molecular weight of the cationic celluloses is in the range from 100,000 to 3,000,000.

Preferable cationic starches are those represented by the following formula (20): 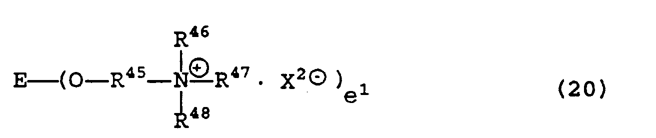 wherein E represents a starch residue, R⁴⁵ represents C_{1-1O} alkylene or C₁₋₁₀ hydroxyalkylene, R⁴⁶, R⁴⁷, and R⁴⁸ are the same or different from each other and represent alkyl, aryl (phenyl), or aralkyl which have carbon atoms of 10 or less and which may form a heterocyclic ring with the nitrogen atom of the formula, and X² represents an anion such as a chloride, bromide, iodide, a sulfuric ion, a sulfonic ion, a methylsulfuric ion, a phosphoric ion, a nitric ion, etc., and e¹ denotes a positive number.

The cationic substitution degree of these cationic starches, or the number of cationic groups introduced per unit of anhydrous glucose, is preferably from 0.01 to 1 and more preferably from 0.02 to 0.5. Cationic substitution degrees of less than 0.01 are just insufficient. Whereas, although the cationic substitution degree may be greater than 1, degrees less than or equal to 1 are particularly preferred in view of the yield of the reaction.

Preferable cationic guar gums are those represented by the following formula (21): 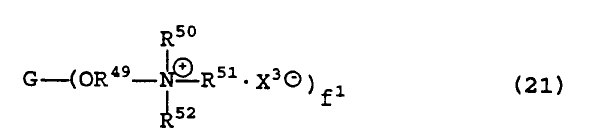 wherein G represents a guar gum residue, R⁴⁹ represents C₁₋₁₀ alkylene or C₁₋₁₀ hydroxyalkylene, R⁵⁰, R⁵¹, and R⁵² are the same or different from each other and represent alkyl, aryl (phenyl), or aralkyl which have carbon atoms of 10 or less and which may form a heterocyclic ring with the nitrogen atom of the formula, and X³ represents an anion such as chloride, bromide, iodide, a sulfuric ion, a sulfonic ion, a methylsulfuric ion, a phosphoric ion, a nitric ion, etc., and f¹ denotes a positive number.

The cationic substitution degree of these cationic guar gums is preferably from 0.01 to 1, per saccharide unit. Especially, those in which 0.02 to 0.5 cationic groups are introduced into the saccharide unit are preferred. Such cationic polymers are described, for example, in Japanese Patent Publication (kokoku) No. 58-35640, Japanese Patent Publication (kokoku) No. 60-46158, and Japanese Patent Application Laid-open (kokai) No. 58-53996. They are commercially available under the trademark "JAGUAR" (Cellanese Schtein Hall Co.).

Preferable examples of the cationic diallyl quaternary ammonium salt/acrylic amide copolymers include those represented by the following formulae (22) or (23): 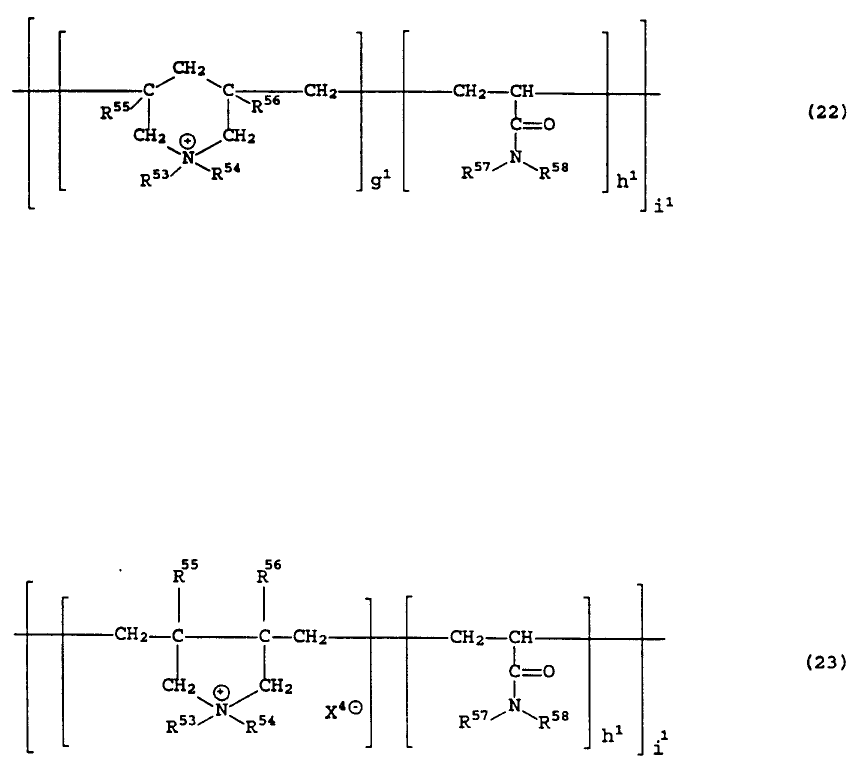 wherein R⁵³ and R⁵⁴ are the same or different from each other and represent hydrogen, alkyl (C₁₋₁₈), phenyl, aryl (tolyl, xylyl), hydroxy C₁₋₁₈-alkyl, amide C₁₋₁₈-alkyl, cyano C₁₋₁₈-alkyl, alkoxy C₁₋₁₈-alkyl, or carboalkoxy C₁₋₁₈-alkyl, R⁵⁵, R⁵⁶, R⁵⁷, and R⁵⁸ are the same or different from each other and each represents hydrogen, lower alkyl (C₁₋₃), or phenyl, and X⁴ represents an anion such as a chloride, bromide, iodide, a sulfuric ion, a sulfonic ion, a methylsulfuric ion, a nitric ion, etc., g¹ represents a number from 1 to 50 inclusive, h¹ represents a number from 0 to 50 inclusive, and i¹ represents a number from 150 to 8,000 inclusive.

The number average molecular weight of these diallyl quaternary ammonium salt/acrylic amide copolymers and diallyl quaternary ammonium salt homopolymers is preferably from about 30,000 to 2,000,000 and particularly preferably from 100,000 to 1,000,000.

Preferable examples of the quaternarized polyvinyl pyrrolidones include those represented by the following formula (24): 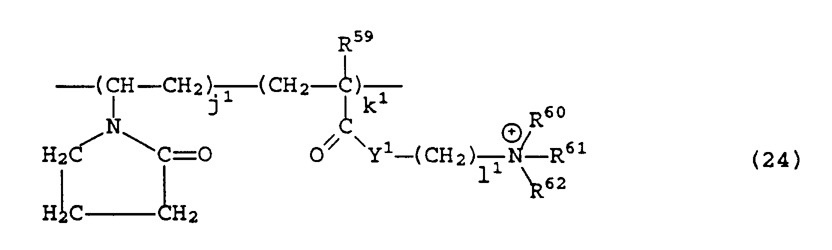 wherein R⁵⁹ represents hydrogen or C₁₋₃ alkyl, R⁶⁰, R⁶¹, and R⁶² are the same or different from each other and each represents hydrogen, C₁₋₁₈ alkyl, hydroxy C₁₋₁₈-alkyl, amide C₁₋₁₈-alkyl, cyano C₁₋₁₈-alkyl, alkoxy C₁₋₁₈-alkyl, or carboalkoxy C₁₋₁₈-alkyl, Y¹ represents oxygen or a group NH in an amide bond, X⁵ represents an anion such as chloride, bromide, iodide, a sulfuric ion, a sulfonic ion, an alkyl sulfuric ion having 1 to 4 carbon atoms, phosphoric ion, and nitric ion, etc., i¹ represents a number from 1 to 10 inclusive, and j¹ and k¹ are numbers making the sum, j¹ + k¹, from 20 to 8,000.

The number average molecular weight of these quaternarized polyvinylpyrrolidones is preferably from 10,000 to 2,000,000 and particularly preferably from 50,000 to 1,500,000.

These water-soluble cationic polymers of component (B) can be used singly or in combinations of two or more. When they are incorporated in an amount from 0.01 to 5% by weight, preferably from 0.1 to 3%, more preferably from 0.3 to 1.5% by weight, based on the total weight of the hair cosmetic compositions of the present invention, excellent conditioning effects can be obtained. The ratio by weight of the component (A) to component (B), i.e., [(A)/(B)] is preferably from 1000/1 to 5/1 and more preferably from 100/1 to 10/1 from the viewpoint of good conditioning effects.

The hair cosmetic compositions of the present invention may further contain, in addition to the above-described essential components, other optional components which are ordinarily used in the manufacture of cosmetics, medicines, and foods. Such optional components include surfactants such as cationic surfactants, anionic surfactants, amphoteric surfactants, and nonionic surfactants; higher alcohols having linear or branched alkyl or alkenyl; hydrocarbons such as liquid paraffin and Vaseline; lanolin derivatives such as liquid lanolin and lanolin fatty acids; phospholipids such as lecithin; sterols such as cholesterol and their derivatives; collagen decomposing peptide derivatives; perfluoropolyethers; oils and fats such as higher alcohol higher fatty acid esters, higher fatty acids, long-chain amide amines having alkyl or alkenyl groups; animal or vegetable oils and fats such as mink oil and olive oil; pharmaceutical agents such as anti-dandruffs typified by zinc pyrrithione (Zpt), germicides, and vitamins; preservatives such as parabene; humectants such as propylene glycol, glycerol, diethylene glycol monoethyl ether, sorbitol, pantenol, and glycinebetaine; coloring agents such as dyes and pigments; conditioning agents such as perfluoro polyethers; pearl-hue imparting agents such as glycol esters; chitosan derivatives such as hydroxypropyl chitosan; blended perfumes of various kinds; and other ingredients described in "ENCYCLOPEDIA OF SHAMPOO INGREDIENTS" (Micelle Press, 1985) or "New Cosmeticology" (Yakujinippou Co., 1988) as long as they do not impede the effects of the present invention.

The hair cosmetic compositions of the present invention can be prepared by known methods by blending the above components. Preferably, the hair cosmetic compositions of the invention are hair detergent compositions such as shampoos, and hair treatment compositions including hair rinses and hair conditioners.

When shampoos are manufactured, surfactants (especially, anionic surfactants, nonionic surfactants or amphoteric surfactants) are incorporated preferably in amounts from 5 to 30% by weight, based on the total weight of the composition, in addition to the aforementioned essential components (A) and (B). Moreover, the hair cosmetic compositions of the present invention are preferably aqueous shampoo compositions or aqueous hair treatment compositions containing water as well as the essential components (A) and (B). Water is incorporated in an amount of 20 to 95%, based on the total weight of the composition.

The hair care compositions of the present invention typically have a pH of 4 to 10, preferably 6 to 8.

The hair cosmetic compositions of the present invention have excellent stability, and during rinsing, the components (A) and (B) cohere to each other and remain on the hair fibers thereby exhibiting excellent conditioning effects. Therefore they are suitable as hair shampoos, hair rinses, hair conditioners, hair treatments, and similar products.

The present invention also provides a method for washing or conditioning hair. The present method involves applying a hair care composition according to the present invention to hair, preferably wet hair, for a time to effect the desired washing or conditioning and then rinsing the hair with water.

Other features of the invention will become apparent in the course of the following descriptions of exemplary embodiments which are given for illustration of the invention and are not intended to be limiting thereof.

### EXAMPLES

### Preparation Example 1:

A solution of phosphorus oxychloride (15.2 g) in tetrahydrofuran (15 g) was cooled to -30°C. To this solution, a solution of two-terminal alcohol-modified dimethylpolysiloxane (50 g) (trademark: X-22-160AS, alcohol equivalent = 112, product of Shin'etsu Kagaku) represented by the following formula: 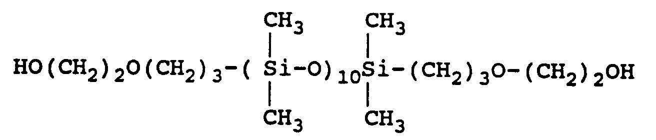 and triethylamine (10 g) in tetrahydrofuran (40 g) was added dropwise over 40 minutes to cause a reaction. Thereafter, the reaction system was maintained at a temperature not higher than -20°C, and the reaction was allowed to cure for 4 hours. Subsequently, a solution of NaOH (20.7 g) in ion exchanged water (40 g) was added to the resulting cured solution over 40 minutes. The mixture was stirred at 0°C for 12 hours to complete the reaction. The solvent was distilled off, and ion exchanged water (300 g) was added to the residue. The insoluble matter was removed by filtration. The filtrate was combined with ethanol (100 ml). The solid matter generated was removed by filtration, to obtain 52 g of a phosphate-modified dimethylpolysiloxane (Compound 1).

This compound was subjected to NMR and IR (KBr pellet method) analyses. The results of the NMR analysis are as follows:
³¹P-NMR (CHCl₃):
   δ(ppm) 8.2 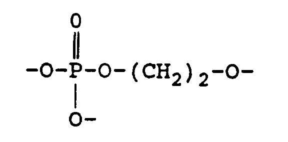
1H - NMR (D₂O):
δ (ppm) 0.1 (broad s, 6.6 H, Si-CH₃) 0.52 (m, 4H, -O-Si-CH₂-CH₂-) 1.55 (m, 4H, -O-Si-CH₂-CH₂-CH₂-O-) 3.6 (m, 8H, -CH₂-O-CH₂-) 3.8 (m, 4H, -CH₂-O-P-)

From the above data, the phosphate-modified dimethylpolysiloxane (Compound 1) is found to have the following structure.

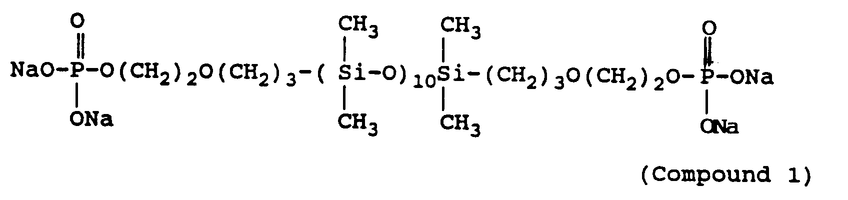

### Preparation Examples 2 to 16:

The procedure of Preparation Example 1 was repeated to obtain the following compounds (Compounds 2 to 16).

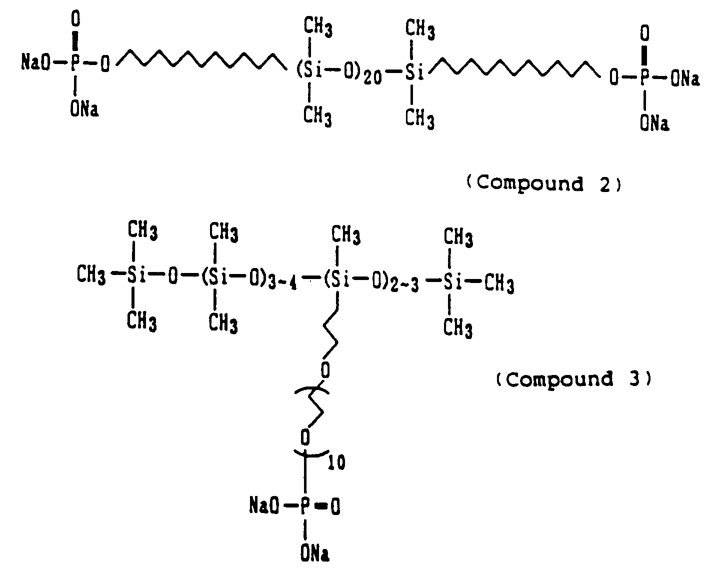

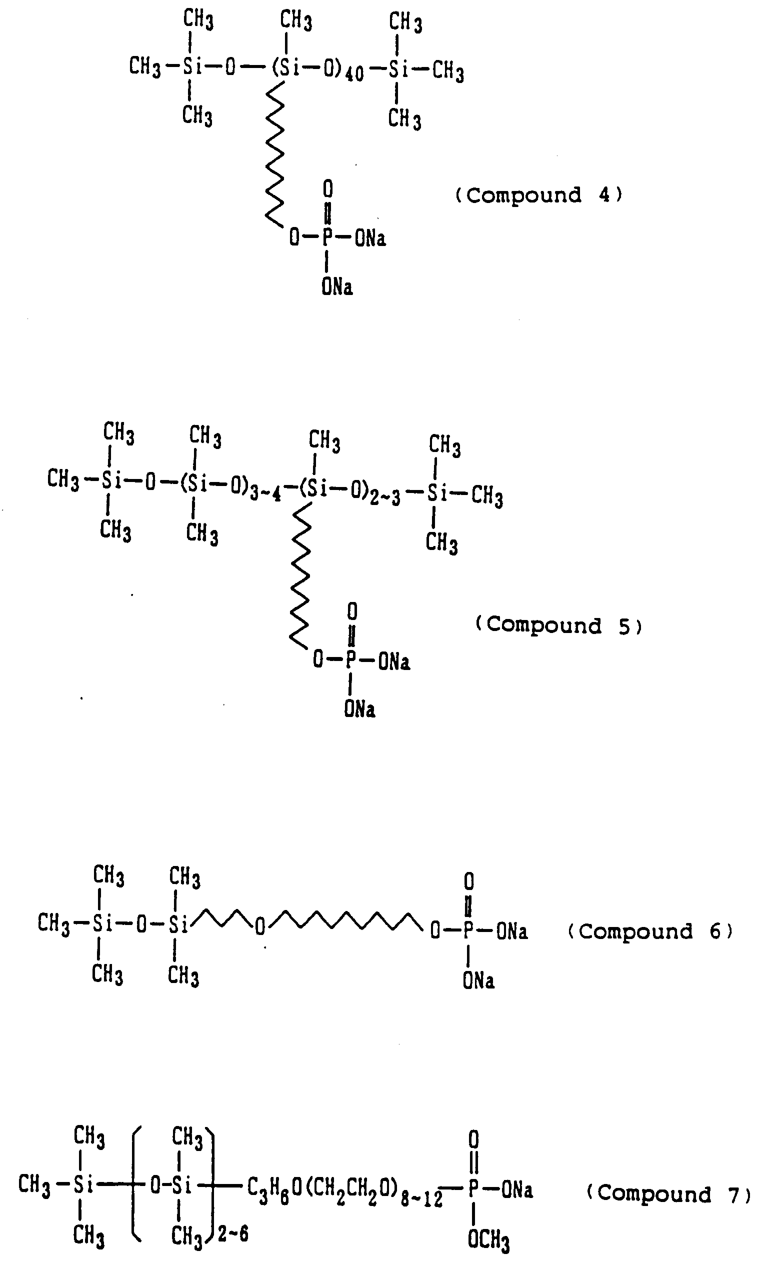

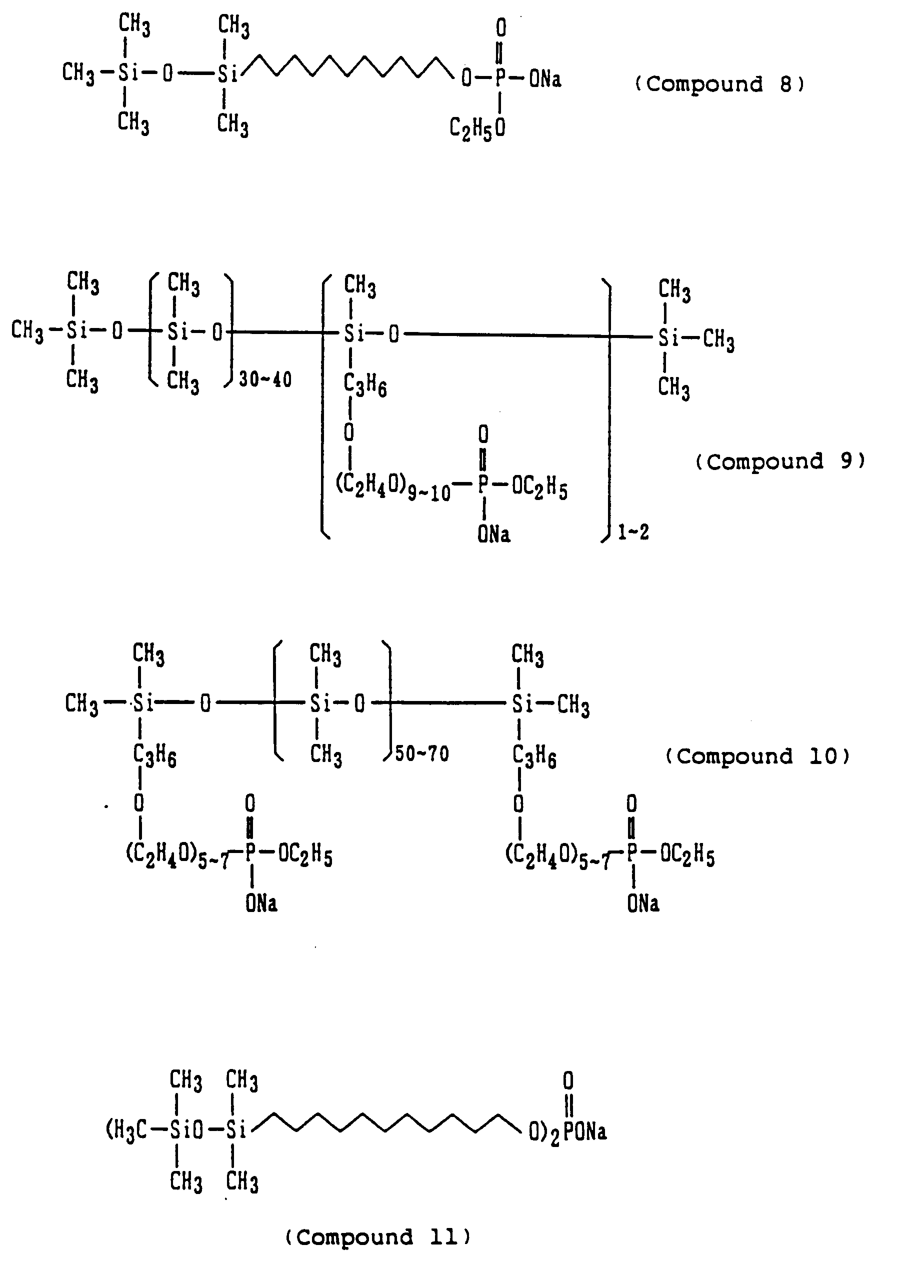

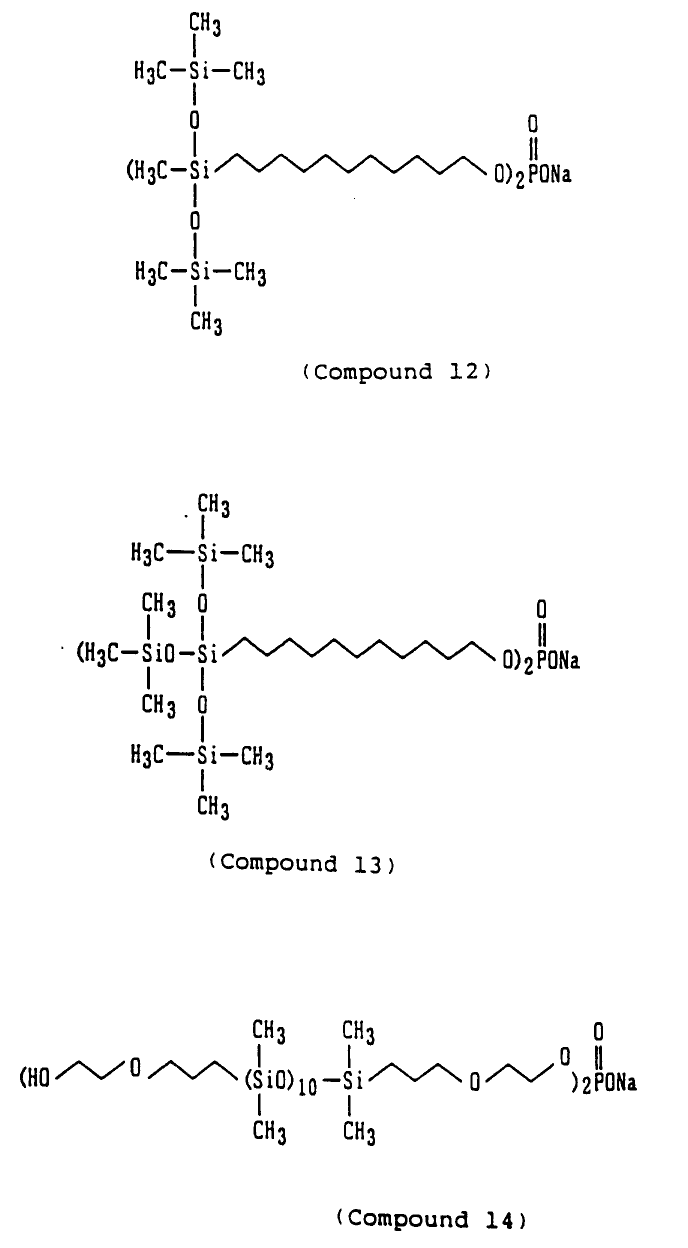

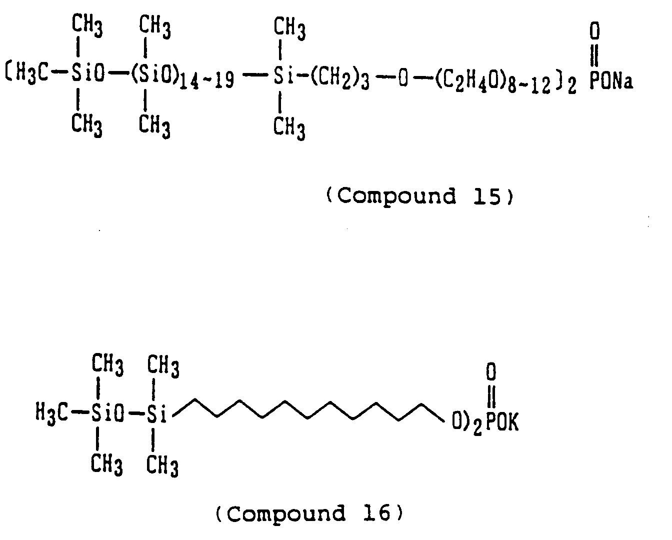

### Preparation Example 17:

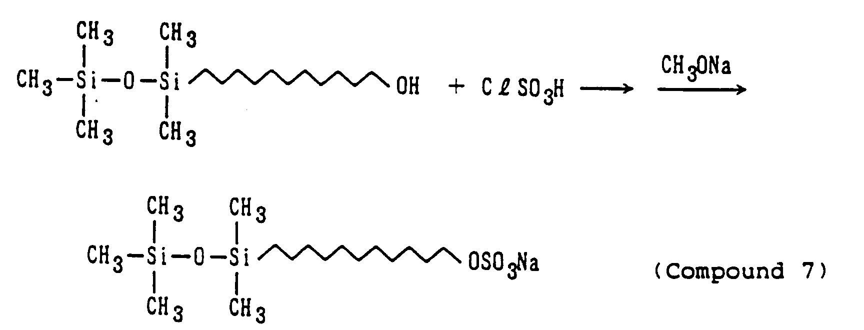

A one-terminal alcohol-modified organosiloxane (15 g) synthesized by a known method, pyridine (7-5 g), and methylene chloride (10 g) were mixed and cooled to 0°C. To this solution, a solution of chlorosulfonic acid (5.5 g) and methylene chloride (10 g) was added dropwise over 20 minutes at a temperature not higher than 10°C to cause a reaction. Thereafter, the reaction system was maintained at a temperature not higher than 10°C, and the reaction was allowed to cure for 4 hours. Subsequently, sodium methylate (18.2 g, 28% methanol solution) was added to the resulting cured solution over 40 minutes. The mixture was stirred at a temperature not higher than 10°C for 15 minutes. The reaction temperature was then gradually raised, and the reaction was stirred at room temperature for 2 hours. When the stirring was completed, the solvent was distilled under reduced pressure. Chloroform was added to the residue, and the insoluble matter was removed by filtration. The solvent was distilled, and the residue was combined with hexane (100 ml). The solid matter generated was collected by filtration, obtaining 17 g of a sulfate-modified organosiloxane (compound 7).

### Preparation Example 18:

The procedure of Preparation Example 17 was repeated to obtain the following sulfate-modified organosiloxanes (Compounds 18 to 30). 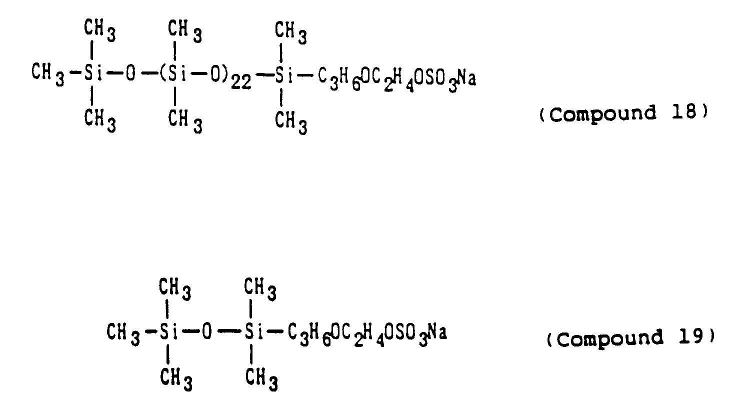 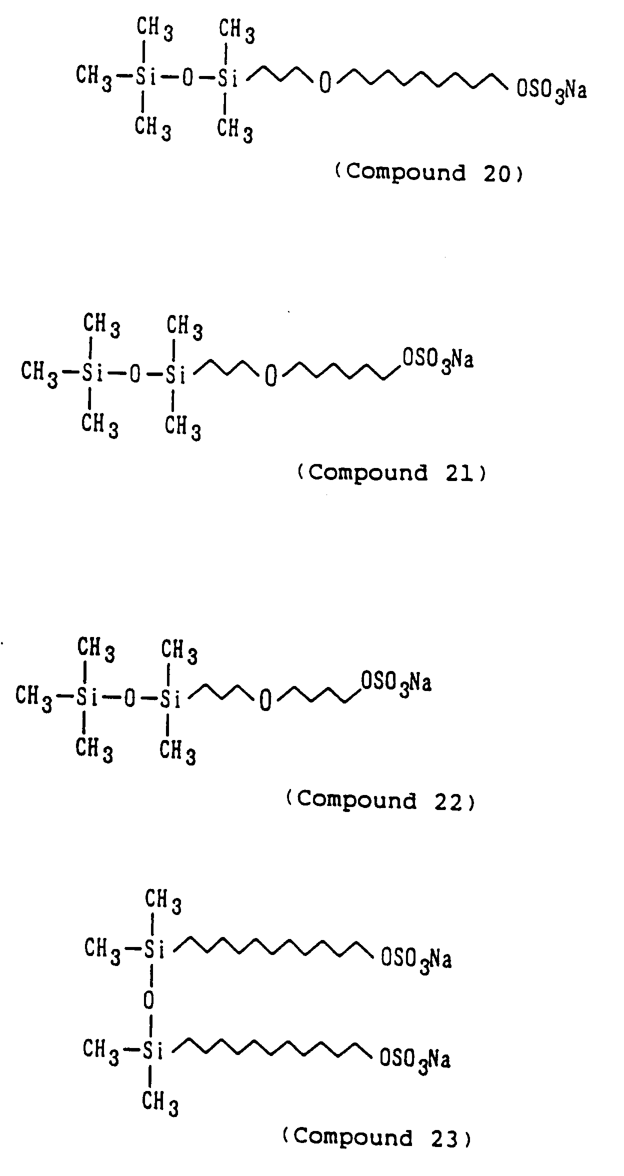 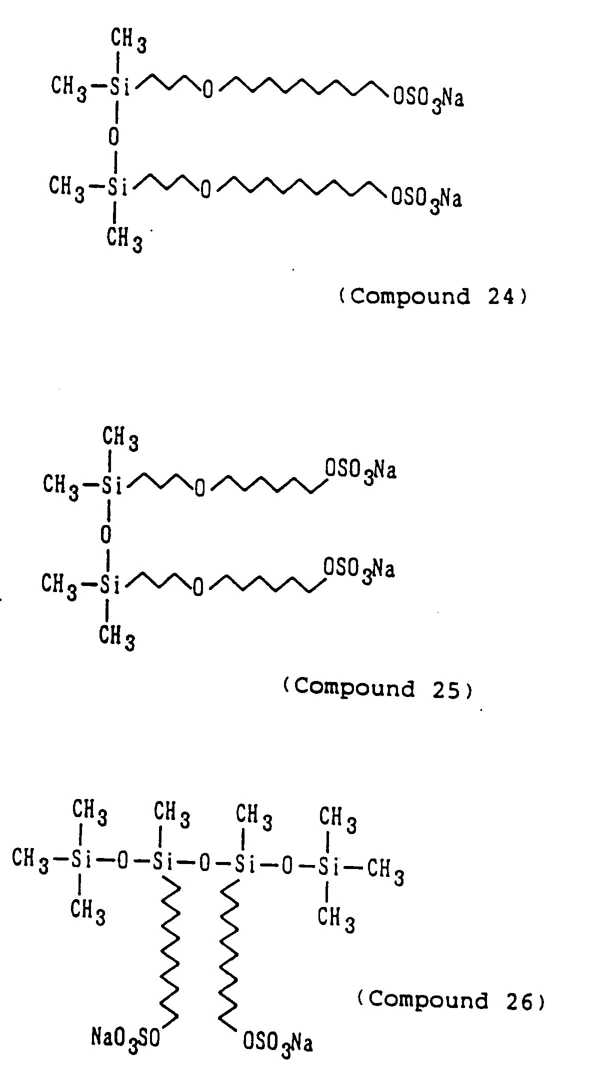 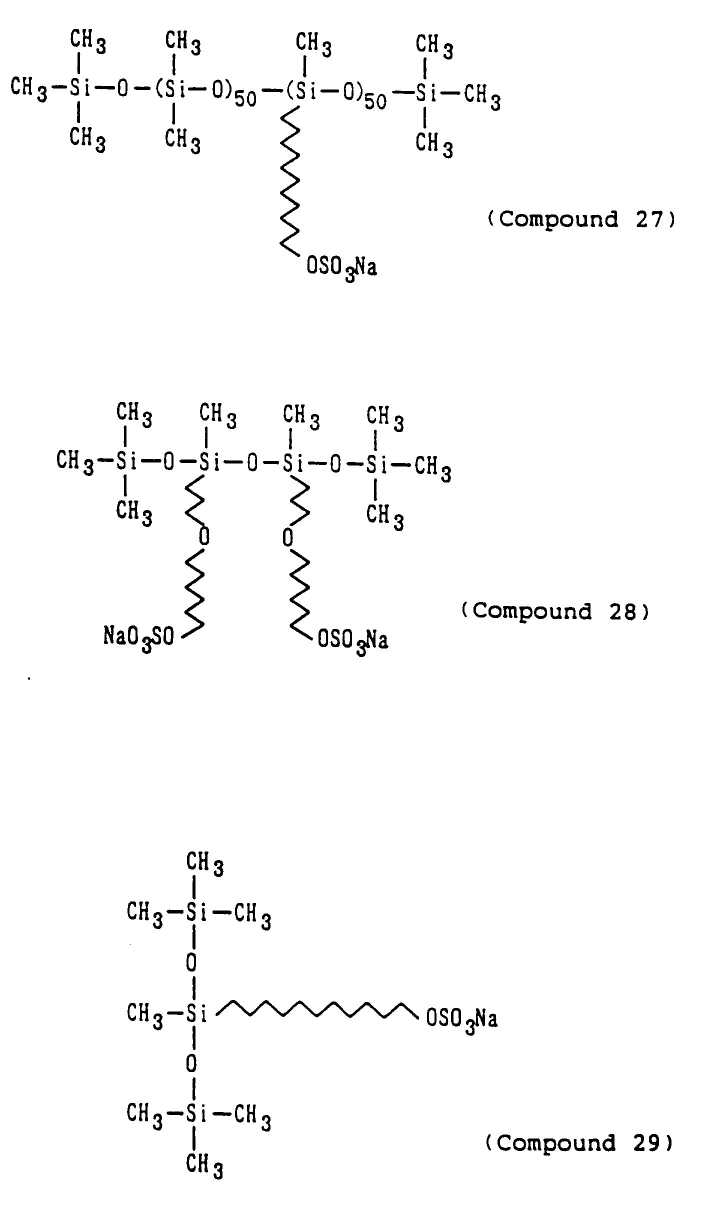 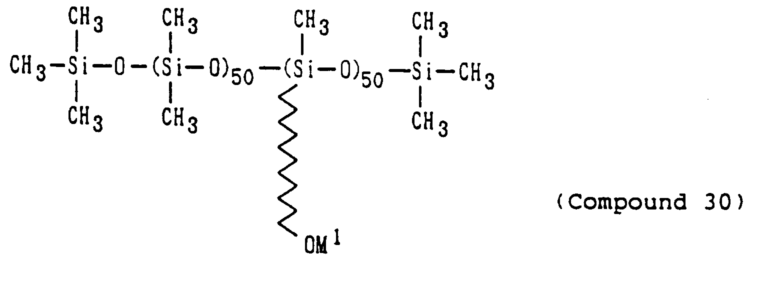 wherein 50 groups of -OM¹ are present as mixtures of -OH and -OSO₃Na.

### Example 1:

The hair cosmetic compositions (shampoos) shown in Table 1 were prepared, and their stability and combing smoothness when the hair was dried after use of the compositions were evaluated. The results are also shown in Table 1.

### Evaluation Method:

### Stability:

The hair cosmetic compositions prepared were allowed to stand for 10 days at room temperature. Thereafter, they were visually checked according to the following criteria.
O: uniform solution
X: separated into 2 phases

### Combing smoothness after drying:

The hair cosmetic compositions were applied to the hair, and then rinsed with water. The hair was dried with a drier. The combing smoothness was evaluated according to the following criteria.
A: very smoothly combed
B: smoothly combed
C: hair fibers are slightly caught by the comb
D: hair fibers, especially tip portions, are caught by the comb

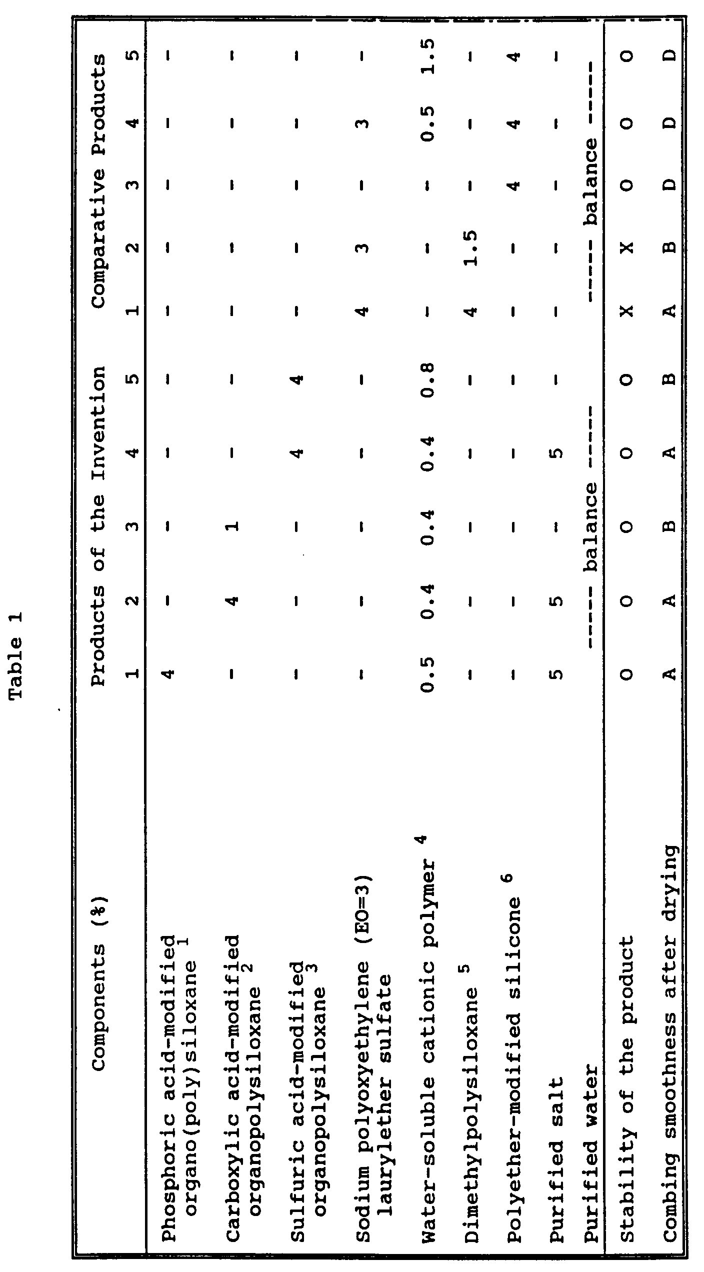

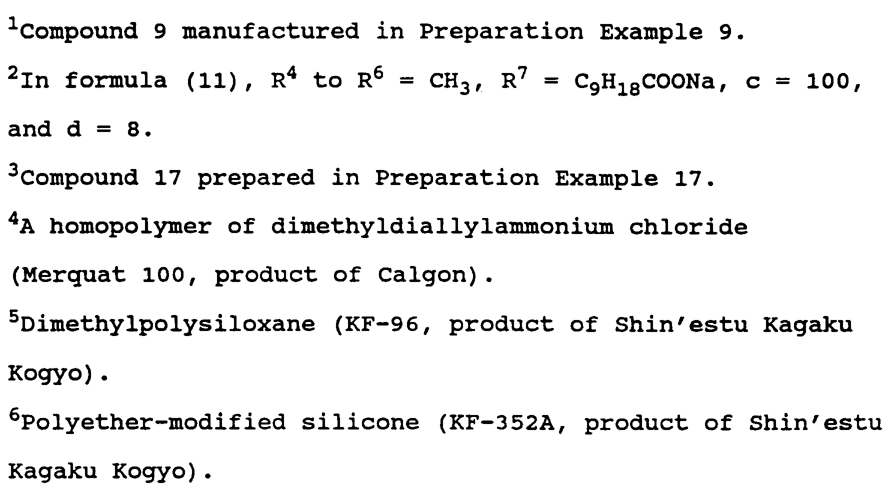

From the data in Table 1, it is understood that the hair cosmetic compositions of the present invention have excellent stability and impart excellent combing smoothness (conditioning effect) to hair fibers.

### Example 2:

A shampoo composition having the following formulation was prepared in a conventional manner. This shampoo product had excellent stability and remarkable conditioning effects.

| (Formulation) | wt% |
|---|---|
| Alkylpolyglycoside [RO(R'O)ₓ,G_{y'}:R = C₈₋₁₂, G = glucose unit, x' = 0, y' = 1 to 3] | 15 |
| Phosphoric acid-modified organo(poly)siloxane⁷ | 3 |
| Water-soluble cationic polymer⁸ | 0.5 |
| Purified salt | 4 |
| Preservative | suitable amount |
| Colorant | suitable amount |
| Perfume | suitable amount |
| Purified water | balance |
| | 100.0 |

| | |
|---|---|
| 7): Compound 7 prepared in Preparation Example 7. | |
| 8): A homopolymer of dimethyldiallylammonium chloride (Merquat 100, product of Calgon). | |

### Example 3:

The procedure of Example 2 was repeated using Compounds 8 to 10 prepared in Preparation Examples 8 to 10 instead of using the phosphoric acid-modified organo(poly)siloxane to prepare shampoo compositions.

All the obtained compositions had excellent stability and exhibited remarkable conditioning effects.

### Example 4:

A hair cosmetic composition having the following formulation was prepared in a conventional manner. This product had excellent stability and exhibited remarkable conditioning effects.

| (Formulation) | wt% |
|---|---|
| Phosphoric acid-modified organo(poly)siloxane⁹ | 4 |
| Water-soluble cationic polymer¹⁰ | 0.5 |
| Sodium polyoxyethylene(3)lauryl sulfate | 3 |
| Polyether-modified silicone¹¹ | 1 |
| Purified salt | 2 |
| Preservative | suitable amount |
| Colorant | suitable amount |
| Perfume | suitable amount |
| Purified water | balance |
| | 100.0 |

| | |
|---|---|
| ⁹Compound 1 prepared in Preparation Example 1. | |
| ¹⁰Cationic cellulose (Polymer JR-400, product of Union Carbide). | |
| ¹¹KF-351A (product of Shin'etsu Kagaku Kogyo). | |

### Example 5:

The procedure of Example 4 was repeated using Compounds 4 to 6 prepared in Preparation Examples 4 to 6 instead of using the phosphoric acid-modified organo(poly)siloxane to prepare hair cosmetic compositions.

All the obtained compositions had excellent stability and exhibited remarkable conditioning effects.

### Example 6:

A hair cosmetic composition having the following formulation was prepared in a conventional manner.

The obtained composition had excellent stability and exhibited remarkable conditioning effects. Especially, it exhibited excellent combing smoothness after the hair was dried.

| (Formulation) | wt% |
|---|---|
| Phosphoric acid-modified organo(poly)siloxane¹² | 4 |
| Water-soluble cationic polymer¹³ | 0.5 |
| Sodium polyoxyethylene(3)lauryl sulfate | 3 |
| Dimethylpolysiloxane¹⁴ | 1 |
| Purified salt | 2 |
| Preservative | suitable amount |
| Colorant | suitable amount |
| Perfume | suitable amount |
| Purified water | balance |
| | 100.0 |

| | |
|---|---|
| ¹²Compound 2 prepared in Preparation Example 2. | |
| ¹³A copolymer of dimethyldiallylammonium chloride and acrylic amide (Merquat 550, product of Calgon). | |
| ¹⁴KF-96 (product of Shin'etsu Kagaku Kogyo). | |

### Example 7:

The procedure of Example 6 was repeated using Compound 3 prepared in Preparation Example 3 instead of using the phosphoric acid-modified organo(poly)siloxane to prepare a hair cosmetic composition.

The obtained composition had excellent stability and exhibited remarkable conditioning effects. Especially, it exhibited excellent combing smoothness when the hair was dried.

### Example 8:

A shampoo composition having the following formulation was prepared in a conventional manner.

The obtained shampoo had excellent lather forming ability and stability and exhibited remarkable conditioning effects.

| (Formulation) | wt% |
|---|---|
| Phosphoric acid-modified organo(poly)siloxane¹⁵ | 3 |
| Water-soluble cationic polymer¹⁶ | 0.5 |
| Alkylsaccharide | 15 |
| Dimethylpolysiloxane¹⁷ | 1 |
| Purified salt | 3 |
| Preservative | suitable amount |
| Colorant | suitable amount |
| Perfume | suitable amount |
| Purified water | balance |
| | 100.0 |

| | |
|---|---|
| ¹⁵Compound 11 prepared in Preparation Example 11. | |
| ¹⁶Cationic cellulose (H-60, product of Kao). | |
| ¹⁷KF-96 (product of Shin'etsu Kagaku Kogyo). | |

### Example 9:

The procedure of Example 8 was repeated using Compounds 12 to 16 prepared in Preparation Examples 12 to 16 instead of using the phosphoric acid-modified organo(poly)siloxane to prepare shampoo compositions.

All the obtained shampoos had excellent lather forming ability and stability and exhibited remarkable conditioning effects.

### Example 10:

A shampoo composition having the following formulation was prepared in a conventional manner.

The obtained composition had excellent stability and exhibited remarkable conditioning effects.

| (Formulation) | wt% |
|---|---|
| Alkylpolyglycoside [RO(R'O)ₓ,G_{y}' : R = C₁₀-alkyl, G = glucose unit, x' = 0, y' = 1.2] | 15 |
| Carboxylic acid-modified organo(poly)siloxane¹⁸ | 3 |
| Water-soluble cationic polymer¹⁹ | 0.5 |
| Purified salt | 4 |
| Preservative | suitable amount |
| Colorant | suitable amount |
| Perfume | suitable amount |
| Purified water | balance |
| | 100.0 |

| | |
|---|---|
| ¹⁸In formula (11), c = d = 0, R⁶ = CH₃, R⁵ = two CH₃'s and one C₃H₁₈COONa. | |
| ¹⁹A homopolymer of dimethyldiallylammonium chloride (Merquat 100, product of Calgon). | |

### Example 11:

A hair cosmetic composition having the following formulation was prepared in a conventional manner.

The obtained composition had excellent stability and exhibited remarkable conditioning effects.

| (Formulation) | wt% |
|---|---|
| Carboxylic acid-modified organo(poly)siloxane²⁰ | 4 |
| Water-soluble cationic polymer²¹ | 0.5 |
| Sodium polyoxyethylene(3)lauryl sulfate | 3 |
| Polyether-modified silicone²² | 1 |
| Purified salt | 2 |
| Preservative | suitable amount |
| Colorant | suitable amount |
| Perfume | suitable amount |
| Purified water | balance |
| | 100.0 |

| | |
|---|---|
| ²⁰In formula (11), R⁷ = C₉H₁₈COONa, R⁴ to R⁶ = CH₃, and x = y = 50. | |
| ²¹Cationic cellulose (Polymer JR-400, product of Union Carbide). | |
| ²²KF-351A (product of Shin'estu Kagaku Kogyo). | |

### Example 12:

A hair cosmetic composition having the following formulation was prepared in a conventional manner.

The obtained composition had excellent stability and exhibited remarkable conditioning effects. Especially, it exhibited excellent combing smoothness when the hair was dried.

| (Formulation) | wt% |
|---|---|
| Carboxylic acid-modified organo(poly)siloxane²³ | 4 |
| Water-soluble cationic polymer²⁴ | 0.2 |
| Sodium polyethylene(3)lauryl sulfate | 3 |
| Dimethylpolysiloxane²⁵ | 1 |
| Purified salt | 2 |
| Preservative | suitable amount |
| Colorant | suitable amount |
| Perfume | suitable amount |
| Purified water | balance |
| | 100.0 |

| | |
|---|---|
| ²³In formula (11), x = y = 0, R⁵ = CH₃, R⁶ = two CH₃'s and one COO(TEA). | |
| ²⁴A copolymer of dimethyldiallylammonium chloride and acrylic amide (Merquat 550, product of Calgon). | |
| ²⁵KF-96 (product of Shin'etsu Kagaku Kogyo). | |

### Example 13:

A shampoo composition having the following formulation was prepared in a conventional manner.

The obtained shampoo composition had excellent lather producing ability and stability, and exhibited remarkable conditioning effects.

| (Formulation) | wt% |
|---|---|
| Carboxylic acid-modified organo(poly)siloxane²⁶ | 3 |
| Cationic polymer²⁷ | 0.3 |
| Alkylsaccharide | 15 |
| Dimethylpolysiloxane²⁸ | 1 |
| Purified salt | 3 |
| Preservative | suitable amount |
| Colorant | suitable amount |
| Perfume | suitable amount |
| Purified water | balance |
| | 100.0 |

| | |
|---|---|
| ²⁶In formula (11), x = y = 0, R⁵ = CH₃, R⁶ = two CH₃'s and one C₉H₁₈COONa. | |
| ²⁷Cationic cellulose (H-60, product of Kao). | |
| ²⁸KF-96 (product of Shin'estu Kagaku Kogyo). | |

### Example 14:

A shampoo composition having the following formulation was prepared in a conventional manner.

The obtained composition had excellent stability and exhibited remarkable conditioning effects.

| (Formulation) | wt% |
|---|---|
| Alkylpolyglycoside [RO(R'O)x,G_{y}': R = C₁₀-alkyl; G = glucose unit, x' = 0, y = 1.21 | 15 |
| sulfuric acid-modified organo(poly)siloxane²⁹ | 3 |
| water-soluble cationic polymer³⁰ | 0.5 |
| Purified salt | 4 |
| Preservative | suitable amount |
| Colorant | suitable amount |
| Perfume | suitable amount |
| Purified water | balance |
| | 100.0 |

| | |
|---|---|
| ²⁹Compound 17 prepared in Preparation Example 17. | |
| ³⁰A homopolymer of dimethyldiallylammonium chloride (Merquat 100, product of Calgon). | |

### Example 15:

The procedure of Example 14 was repeated using Compounds 18 to 24 prepared in Preparation Example 18 instead of using the sulfuric acid-modified organo(poly)siloxane to prepare shampoo compositions.

All the obtained compositions had excellent stability and exhibited remarkable conditioning effects.

### Example 16:

A hair cosmetic composition having the following formulation was prepared in a conventional manner.

The obtained Composition had excellent stability and exhibited remarkable conditioning effects.

| (Formulation) | wt% |
|---|---|
| Sulfuric acid-modified organo(poly)siloxane³¹ | 4 |
| Water-soluble cationic polymer³² | 0.5 |
| Sodium polyoxyethylene(3)lauryl sulfate | 3 |
| Polyether-modified silicone³³ | 1 |
| Purified salt | 2 |
| Preservative | suitable amount |
| Colorant | suitable amount |
| Perfume | suitable amount |
| Purified water | balance |
| | 100.0 |

| | |
|---|---|
| ³¹Compound 17 prepared in Preparation Example 17. | |
| ³²Cationic cellulose (Polymer JR-400, product of Union Carbide). | |
| ³³KF-351A (product of Shin'estu Kagaku Kogyo). | |

### Example 17:

The procedure of Example 16 was repeated using Compounds 18 to 30 prepared in Preparation Example 18 instead of using the sulfuric acid-modified organo(poly)siloxane to prepare shampoo compositions.

All the obtained compositions had excellent stability and exhibited remarkable conditioning effects.

### Example 18:

A hair cosmetic composition having the following formulation was prepared in a conventional manner.

The obtained composition had excellent stability and exhibited remarkable conditioning effects. Especially it exhibited excellent combing smoothness when the hair was dried.

| (Formulation) | wt% |
|---|---|
| Sulfuric acid-modified organo(poly)siloxane³² | 4 |
| Water-soluble cationic polymer³³ | 0.3 |
| Sodium polyethylene(3)lauryl sulfate | 3 |
| Dimethylpolysiloxane³⁴ | 1 |
| Purified salt | 2 |
| Preservative | suitable amount |
| Colorant | suitable amount |
| Perfume | suitable amount |
| Purified water | balance |
| | 100.0 |

| | |
|---|---|
| ³²Compound 18 prepared in Preparation Example 18. | |
| ³³A copolymer of dimethyldiallylammonium chloride and acrylic amide (Merquat 550, product of Calgon). | |
| ³⁴KF-96 (product of Shin'estu Kagaku Kogyo). | |

### Example 19:

The procedure of Example 18 was repeated using Compound 19 prepared in Preparation Example 18 instead of using the sulfuric acid-modified organo(poly)siloxane to prepare hair cosmetic compositions.

The obtained composition had excellent stability and exhibited remarkable conditioning effects. Especially, it exhibited remarkable combing smoothness when the hair was dried.

### Example 20:

A shampoo composition having the following formulation was prepared in a conventional manner.

The obtained shampoo composition had excellent lather producing ability and stability, and exhibited remarkable conditioning effects.

| (Formulation) | wt% |
|---|---|
| Sulfuric acid-modified organo(poly)siloxane³⁵ | 3 |
| Water-soluble cationic polymer³⁶ | 0.3 |
| Alkylsaccharide | 15 |
| Dimethylpolysiloxane³⁷ | 1 |
| Purified salt | 3 |
| Preservative | suitable amount |
| Colorant | suitable amount |
| Perfume | suitable amount |
| Purified water | balance |
| | 100.0 |

| | |
|---|---|
| ³⁵Compound 29 prepared in Preparation Example 18. | |
| ³⁶Cationic cellulose (H-60, product of Kao). | |
| ³⁷KF-96 (product of Shin'estu Kagaku Kogyo). | |

### Example 21:

The procedure of Example 20 was repeated using Compound 17 prepared in Preparation Example 17 instead of using the sulfuric acid-modified organo(poly)siloxane to prepare a shampoo composition.

The obtained shampoo composition had excellent latherability and stability and exhibited remarkable conditioning effects.

This application is based on Japanese Patent Application Nos. 32315/1994 filed on March 2, 1994 and 304871/1994 filed on December 8, 1994, which are incorporated herein by reference in its entirety.

Obviously, numerous modifications and variations of the present invention are possible in light of the above teachings. It is therefore to be understood that, within the scope of the appended claims, the invention may be practiced otherwise than as specifically described herein.

## Claims

1. A hair cosmetic composition which comprises the following components (A) and (B):
(A) 0.1 to 20% by weight, based on the total weight of said composition, of an organopolysiloxane having at least one structure of an acidic group or a salt thereof in its molecule; and
(B) 0.01 to 5% by weight, based on the total weight of said composition, of a water-soluble cationic polymer.

2. The hair cosmetic composition of Claim 1, wherein said component (A) is an organopolysiloxane having, in its molecule, a structure selected from the group consisting of a phosphoric group, a phosphate group, a carboxylic group, a carboxylate group, a sulfuric group a sulfate group, a sulfonic group, and a sulfonate group.

3. The hair cosmetic composition of Claim 1, wherein the ratio by weight of said component (A) to said component (B), i.e., (A)/(B), is from 1,000/1 to 5/1.

4. The hair cosmetic composition of Claim 1, wherein said component (A) is a phosphoric acid-modified organopolysiloxane having a structural unit in its molecule represented by the following formula (1) or a salt thereof: 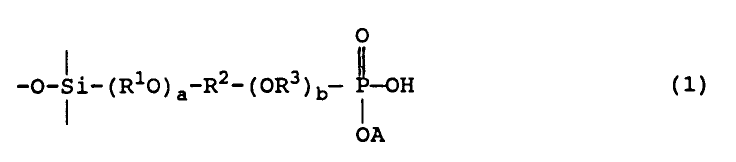 wherein R¹ and R³ each independently represent a C₂₋₂₀ linear or branched alkylene group, R² represents a C₁₋₅₀ linear or branched alkylene group which may have a hydroxyl group, A represents a hydrogen atom, a C₁₋₂₂ linear or branched alkyl or alkenyl group, or the group 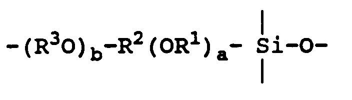 wherein a represents 0 or 1, and b represents a number from 0 to 200 inclusive.

5. The hair cosmetic composition of Claim 1, wherein said component (A) is a carboxylic acid-modified organopolysiloxane having a structural unit in its molecule represented by the following formula (10) or a salt thereof: 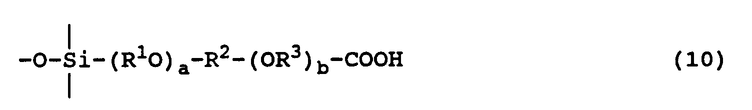 wherein R¹ and R³ each independently represent a C₂₋₂₀ linear or branched alkylene group, R² represents a C₁₋₅₀ linear or branched alkylene group which may have a hydroxyl group, a represents 0 or 1, and b represents a number from 0 to 200 inclusive.

6. The hair cosmetic composition of Claim 1, wherein said component (A) is a sulfuric acid-modified organopolysiloxane having a structural unit in its molecule represented by the following formula (14) or a salt thereof: 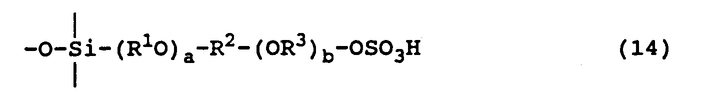 wherein R¹ and R³ each independently represents a C₂₋₂₀ linear or branched alkylene group, R² represents a C₁₋₅₀ linear or branched alkylene group which may have a hydroxyl group, a represents 0 or 1, and b represents a number from 0 to 200 inclusive.

7. The hair cosmetic composition of Claim 1, wherein said component (B) is selected from the group consisting of cationic celluloses, cationic starches, cationic guar gums, homopolymers of diallyl quaternary ammonium salts, diallyl quaternary ammonium salt/acrylic amide copolymers, and quaternary polyvinyl pyrrolidones.

8. The hair cosmetic composition of Claim 1, wherein said composition is a hair rinse, hair treatment, or a hair conditioner.

9. The hair cosmetic composition of Claim 1, which is a shampoo composition further comprising 5 to 30% by weight, based on the total weight of said composition, of a surfactant.

10. A method of washing or conditioning hair, comprising:
(i) applying to hair a composition according to any of claims 1 to 9.
(ii) rinsing said hair.

## Patentansprüche

1. Haarkosmetische Zusammensetzung, umfassend die folgenden Komponenten (A) und (B):
(A) 0,1 bis 20 Gew.%, bezogen auf das Gesamtgewicht der Zusammensetzung, eines Organopolysiloxans mit zumindest einer Struktur einer sauren Gruppe oder eines Salzes davon in dem Molekül; und
(B) 0,01 bis 5 Gew.%, bezogen auf das Gesamtgewicht der Zusammensetzung, eines wasserlöslichen, kationischen Polymers.

2. Haarkosmetische Zusammensetzung nach Anspruch 1, worin die Komponente (A) ein Organopolysiloxan ist, das in seinem Molekül eine Struktur aufweist, ausgewählt aus der Gruppe, bestehend aus einer Phosphorsäuregruppe, einer Phosphatgruppe, einer Carbonsäuregruppe, einer Carboxylatgruppe, einer Schwefelsäuregruppe, einer Sulfatgruppe, einer Sulfonsäuregruppe und einer Sulfonatgruppe.

3. Haarkosmetische Zusammensetzung nach Anspruch 1, worin das Gewichtsverhältnis der Komponente (A) zu der Komponente (B), d.h. (A)/(B), von 1000/1 bis 5/1 ist.

4. Haarkosmetische Zusammensetzung nach Anspruch 1, worin die Komponente (A) ein phosphorsäuremodifiziertes Organopolysiloxan mit einer strukturellen Einheit in seinem Molekül, dargestellt durch die folgende Formel (1), oder ein Salz davon ist: 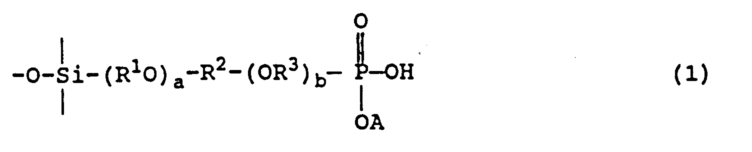 worin R¹ oder R³ jeweils unabhängig eine C₂₋₂₀-lineare oder verzeigte Alkylengruppe, R² eine C₁₋₅₀-lineare oder verzweigte Alkylengruppe, die eine Hydroxylgruppe haben kann, A ein Wasserstoffatom, eine C₁₋₂₂-lineare oder verzweigte Alkyl- oder Alkenylgruppe oder die Gruppe 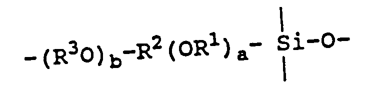 sind, worin a 0 oder 1 und b eine Zahl von 0 bis einschließlich 200 bedeuten.

5. Haarkosmetische Zusammenetzung nach Anspruch 1, worin die Komponente (A) ein carbonsäuremodifiziertes Organopolysiloxan mit einer strukturellen Einheit in seinem Molekül, dargestellt durch die folgende Formel (10), oder ein Salz davon ist: 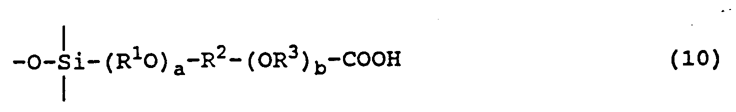 worin R¹ und R³ jeweils unabhängig eine C₂₋₂₀-lineare oder verzweigte Alkylengruppe, R² eine C₁₋₅₀-lineare oder verzweigte Alkylengruppe, die eine Hydroxylgruppe haben kann, a 0 oder 1 und b eine Zahl von 0 bis einschließlich 200 sind.

6. Haarkosmetische Zusammensetzung nach Anspruch 1, worin die Komponente (A) ein schwefelsäuremodifiziertes Organosiloxan mit einer strukturellen Einheit in seinem Molekül, dargestellt durch die folgende Formel (14), oder ein Salz davon ist: 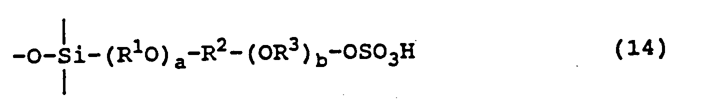 worin R¹ und R³ jeweils unabhängig eine C₂₋₂₀-lineare oder verzweigte Alkylengruppe, R² eine C₁₋₅₀-lineare oder verzweigte Alkylengruppe, die eine Hydroxylgruppe haben kann, a 0 oder 1 und b eine Zahl von 0 bis einschließlich 200 sind.

7. Haarkosmetische Zusammensetzung nach Anspruch 1, worin die Komponente (B) ausgewählt ist aus der Gruppe, bestehend aus kationischen Cellulosen, kationischen Stärken, kationischen Guargummis, Homopolymeren von Diallylquaternären Ammoniumsalzen, Diallyl-quaternäres Ammoniumsalz/Acrylamid-Copolymeren und quaternären Polyvinylpyrrolidonen ist.

8. Haarkosmetische Zusammensetzung nach Anspruch 1, worin die Zusammensetzung eine Haarspülung, Haarbehandlung oder Haarkonditionierer ist.

9. Haarkosmetische Zusammensetzung nach Anspruch 1, die eine Shampoozusammensetzung ist, die weiterhin 5 bis 30 Gew.%, bezogen auf das Gesamtgewicht der Zusammensetzung, eines Tensides enthält.

10. Verfahren zum Waschen oder Konditionieren von Haar, umfassend:
(i) Auftragen einer Zusammensetzung nach einem der Ansprüche 1 bis 9 auf das Haar;
(ii) Spülen des Haares.

## Revendications

1. Composition cosmétique pour le cheveu, qui Comprend les constituants suivants (A) et (B):
(A) 0,1 à 20 % en poids, sur base du poids total de ladite composition, d'un organopolysiloxane présentant au moins une structure d'un groupe acide ou d'un sel de celui-ci dans sa molécule; et
(B) 0,01 à 5 % en poids, sur base du poids total de ladite composition, d'un polymère cationique soluble dans l'eau.

2. Composition cosmétique pour le cheveu selon la revendication 1, dans laquelle ledit constituant (A) est un organopolysiloxane présentant, dans sa molécule, une structure choisie dans leur groupe constitué d'un groupe phosphorique, un groupe phosphate, une groupe carboxylique, un groupe carboxylate, un groupe sulfurique, un groupe sulfate, un groupe sulfonique, et un groupe sulfonate.

3. Composition cosmétique pour le cheveu selon la revendication 1, dans laquelle le rapport en poids dudit constituant (A) audit constituant (B) c'est-à-dire (A)/(B), est de 1000/1 à 5/1.

4. Composition cosmétique pour le cheveu selon la revendication 1, dans laquelle ledit constituant (A) est un organopolysiloxane modifié par acide phosphorique présentant dans sa molécule un motif structurel représenté par la formule (1) suivante, ou un sel de celui-ci : 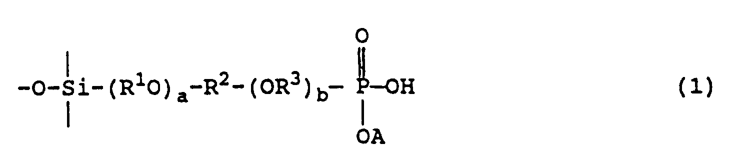 dans laquelle R¹ et R³ représentent chacun indépendamment un groupe alkylène linéaire ou ramifiée en C₂₋₂₀, R² représente un groupe alkylène linéaire ou ramifié en C₁₋₅₀ qui peut posséder un groupe hydroxyle, A représente un atome d'hydrogène, un groupe alcényle ou alkyle linéaire ou ramifié en C₁₋₂₂, ou le groupe 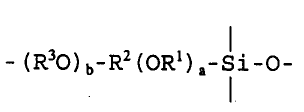 dans lequel a représente 0 ou 1, et b représente un nombre de 0 à 200 inclus.

5. Composition cosmétique pour le cheveu selon la revendication 1, dans laquelle ledit constituant (A) est un organopolysiloxane modifié par acide carboxylique présentant dans sa molécule un motif structurel représenté par la formule (10) suivante, ou un sel de celui-ci : 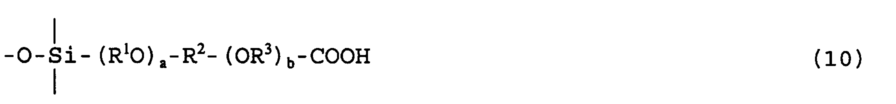 dans laquelle R¹ et R³ représente chacun indépendamment un groupe alkylène linéaire ou ramifié en C₂₋₂₀, R² représente un groupe alkylène linéaire ou ramifié en C₁₋₅₀ qui peut présenter un groupe hydroxyle, a représente 0 ou 1, et b représente un nombre de 0 à 200 inclus.

6. Composition cosmétique pour le cheveu selon la revendication 1, dans laquelle ledit constituant (A) est un organopolysiloxane modifié par acide sulfurique présentant dans sa molécule un motif structurel représenté par la formule (14) suivante ou un sel de celui-ci : 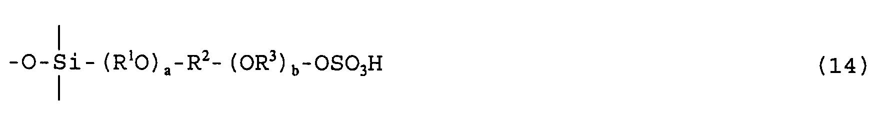 dans laquelle R¹ et R³ représentent chacun indépendamment un groupe alkylène linéaire ou ramifié en C₂₋₂₀, R² représente un groupe alkylène linéaire ou ramifié en C₁₋₅₀ qui peut présenter un groupe hydroxyle, a représente 0 ou 1 et b représente un nombre de 0 à 200 inclus.

7. Composition cosmétique pour le cheveu selon la revendication 1, dans laquelle ledit constituant (B) est choisi dans le groupe constitué de celluloses cationiques, d'amidons cationiques, de gommes arabiques cationiques, d'homopolymères de sels diallyliques d'ammonium quaternaire, de copolymères de sel diallylique d'ammonium quaternaire/amide acrylique, et de pyrrolidones de polyvinyles quaternaires.

8. Composition cosmétique pour le cheveu selon la revendication 1, dans laquelle ladite composition est un produit de rinçage pour le cheveu, un produit de traitement du cheveu, ou un conditionneur du cheveu.

9. Composition cosmétique pour le cheveu selon la revendication 1, qui est une composition de shampooing comprenant en outre 5 à 30 % en poids, sur base du poids total de ladite composition, d'un agent tensioactif.

10. Procédé de lavage ou de conditionnement du cheveu, consistant à :
(i) appliquer une composition pour le cheveu selon l'une quelconque des revendications 1 à 9,
(ii) rincer ledit cheveu.
